# EUROPEAN PATENT SPECIFICATION

(11) **EP 2 318 372 B1**
(45) Date of publication and mention of the grant of the patent: **14.01.2015**
(21) Application number: 09790652.3
(22) Date of filing: 21.07.2009
(51) Int. Cl.: C07D 221/26, A61K 31/485, A61P 25/36, A61P 29/00

(54) **LARGE SUBSTITUENT, NON-PHENOLIC AMINE OPIOIDS**
NICHTPHENOLISCHE AMINOPIOIDE MIT GROSSEM SUBSTITUENTEN
OPIOÏDES AMINÉS NON PHÉNOLIQUES À SUBSTITUANT LARGE

(30) Priority: 21.07.2008 US 82255 P
(43) Date of publication of application: 11.05.2011
(73) Proprietor: Rensselaer Polytechnic Institute, Troy, NY 12180 (US)
(72) Inventor: WENTLAND, Mark P., Menands New York 12204 (US)
(74) Representative: Graf von Stosch, Andreas
(86) International application number: PCT/US2009/051200
(87) International publication number: WO 2010/011619

(56) References cited:
- WO-A-2007/014137
- WENTLAND MARK P ET AL: "Redefining the structure-activity relationships of 2,6-methano-3-benzazocines. 4. Opioid receptor binding properties of 8-[N-(4'-phenyl)-phenethyl)carboxamido] analogues of cyclazocine and ethylketocycalzocine." JOURNAL OF MEDICINAL CHEMISTRY 7 SEP 2006, vol. 49, no. 18, 7 September 2006 (2006-09-07), pages 5635-5639, XP002550099 ISSN: 0022-2623
- VANALSTINE ET AL: "Redefining the structure-activity relationships of 2,6-methano-3-benzazocines. 5. Opioid receptor binding properties of N-((4'-phenyl)-phenethyl) analogues of 8-CAC" BIOORGANIC & MEDICINAL CHEMISTRY LETTERS, PERGAMON, ELSEVIER SCIENCE, GB, vol. 17, no. 23, 2 November 2007 (2007-11-02), pages 6516-6520, XP022325927 ISSN: 0960-894X

## Description

### Field of the Invention

The invention relates to opioid receptor binding compounds containing carboxamides that have large substitutents on the nitrogen of the carboxamide. The compounds are useful as analgesics, anti-diarrheal agents, anticonvulsants, anti-obesity agents, antitussives, anti-cocaine, and anti-addiction medications.

### Background of the Invention

Opiates have been the subject of intense research since the isolation of morphine in 1805, and thousands of compounds having opiate or opiate-like activity have been identified. Many opioid receptor-interactive compounds including those used for producing analgesia (e.g., morphine) and those used for treating drug addiction (e.g., naltrexone and cyclazocine) in humans have limited utility due to poor oral bioavailability and a very rapid clearance rate from the body. This has been shown in many instances to be due to the presence of the 8-hydroxyl group (OH) of 2,6-methano-3-benzazocines, also known as benzomorphans [(e.g., cyclazocine and EKC (ethylketocyclazocine)] and the corresponding 3-OH group in morphinanes (e.g., morphine).

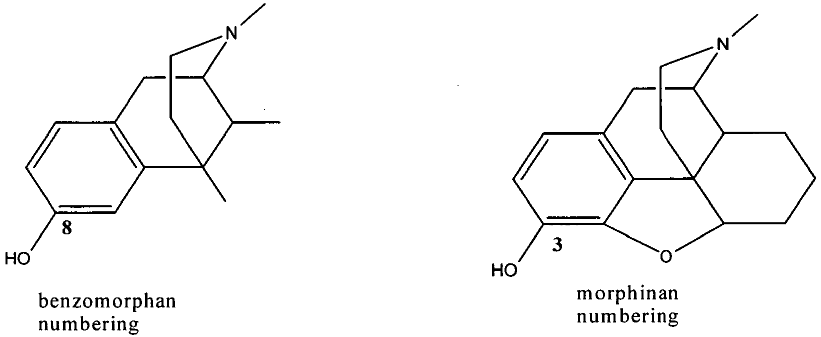

The high polarity of these hydroxyl groups retards oral absorption of the parent molecules. Furthermore, the 8-(or 3-)OH group is prone to sulfonation and glucuronidation (Phase II metabolism), both of which facilitate rapid excretion of the active compounds, leading to disadvantageously short half-lives for the active compounds. Until the publications of Wentland in 2001, the uniform experience in the art of the past seventy years had been that removal or replacement of the 8-(or 3-) OH group had led to pharmacologically inactive compounds.

US patent 6,784,187 (to Wentland) disclosed that the phenolic OH of opioids could be replaced by CONH₂. In the cyclazocine series of opioids, it was shown that 8-carboxamidocyclazocine (8-CAC) had high affinity for µ and κ opioid receptors. In studies in vivo, 8-CAC showed high antinociception activity and a much longer duration of action than cyclazocine (15 h vs. 2 h) when both were dosed at 1 mg/kg ip in mice. Preliminary structure-activity relationship studies for 8-CAC revealed that mono-substitution of the carboxamide nitrogen with methyl or phenyl reduced binding affinity for guinea pig µ receptors 75- and 2313-fold, respectively whereas dimethylation of the carboxamide group reduced binding affinity 9375-fold. The finding that substitution of the carboxamide nitrogen had such a detrimental effect suggested that the NH₂ of the amide was critical to opioid binding.

WO 2007/014137 discloses 8-substituted-2,6-methano-3-benzazocine compounds. The inhibition of the µ opioid receptor, as measured by the Kᵢ value, was observed to be dependent on the Cyclazocine derivative structure.

Wentland et al. (Journal of Medicinal Chemistry, 07 Sept. 2006, vol. 49 (18), pp. 5635-5639) report on structure-activity relationships of 2,6-methano-3-benzazocines, in particular of opioid receptor binding properties of 8-[N-(4'-phenyl)-phenethyl)carboxamido] analogues of cyclazocine and and ethylketocyclazocine. An improved binding affinity to µ, κ, and δ opioid receptors was observed for the H-[N-(4'-phenyl)-phenethyl)carboxamido] analogue.

Vanalstine et al. (Bioorganic & Medicinal Chemistry Letters, Elsevier Science, 02 November 2007, vol. 17 (23), pp. 6516-6520) discuss opioid receptor binding properties of N-((4'-phenyl)-phenethyl)analogues of 8-CAC. The analogues were evaluated to probe a putative hydrophobic binding pocket of opioid receptors. Depending on the substituent, an improved binding affinity to the µ and to the κ opioid receptors was observed.

### Summary of the Invention

We have now found that the nitrogen of the carboxamide can be substituted with fairly large and relatively non-polar groups, and that such compounds exhibit excellent opioid binding and, presumably, good metabolic stability. The compounds of the invention are therefore useful as analgesics, anti-pruritics, anti-diarrheal agents, anticonvulsants, antitussives, anorexics and as treatments for hyperalgesia, drug addiction, respiratory depression, dyskinesia, pain (including neuropathic pain), irritable bowel syndrome and gastrointestinal motility disorders. Drug addiction, as used herein, includes alcohol and nicotine addiction. There is evidence in the literature that the compounds may also be useful as immunosuppressants and antiinflammatories and for reducing ischemic damage (and cardioprotection), for improving learning and memory, and for treating urinary incontinence.

In one aspect, the invention relates to compounds of formula I: 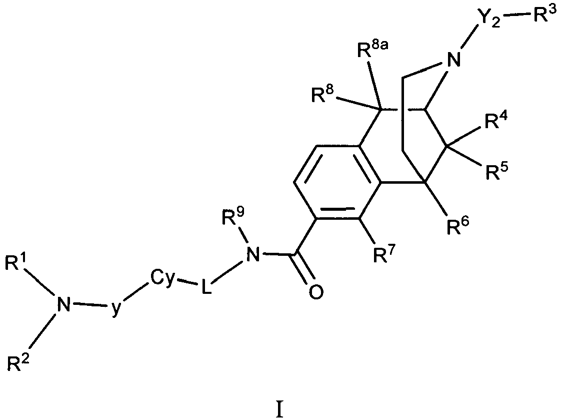 wherein
- R¹ and R²: are each independently chosen from hydrogen and optionally substituted lower alkyl;
- R³: is chosen from hydrogen, C₁-C₈ hydrocarbon, heterocyclyl, aryl and hydroxyalkyl;
- R⁴: is chosen from hydrogen, hydroxyl, C₁-C₂₀ alkyl and C₁-C₂₀ alkyl substituted with hydroxyl or carbonyl;
- R⁵: is lower alkyl;
- R⁶: is lower alkyl;
- R⁷: is chosen from hydrogen, NR¹⁰R¹¹ and -OR¹⁰; or together R⁴, R⁵, R⁶ and R⁷ may form from one to three rings, said rings having optional additional substitution;
- R⁸ and R^{8a}: are both hydrogen or taken together R⁸ and R^{8a} are =O;
- R⁹: is chosen from hydrogen and lower alkyl;
- R¹⁰ and R¹¹: are each independently hydrogen, optionally substituted lower alkyl, optionally substituted alkenyl, optionally substituted alkynyl, optionally substituted aryl, hydroxyl, amino or optionally substituted lower alkoxy;
- y: is -(C(R¹⁰)(R¹¹))p- or a direct bond, wherein p is 0, 1, 2, 3, 4, 5, 6, or 7;
- Y₂: is a direct bond or -(C(R¹⁰)(R¹¹))q-, wherein q is 0, 1, 2, 3, 4 or 5;
- L: is a direct bond or -(C(R¹⁰)(R¹¹))q-; and
- Cy: is Ar¹-B-Ar², wherein Ar¹ is absent, or an aryl or heteroaryl radical having from 1 to 4 N, O and/or S atoms, which may be unsubstituted or mono-, di- or trisubstituted by halogen, lower alkyl, alkenyl, alkynyl, cycloalkyl, -OR¹⁰, -NR¹⁰R¹¹, -CN, -COR¹⁰ or - COOR¹⁰; B is a direct bond, -O-, -NR¹⁰, -SO₂, or -(C(R¹⁰)(R¹¹))s-, wherein s is 0, 1, 2, 3, 4 or 5; and
- Ar²: is aryl or heteroaryl radical having from 1 to 4 N, O and/or S atoms, which may be unsubstituted or mono-, di- or trisubstituted by halogen, lower alkyl, alkenyl, alkynyl, cycloalkyl, -OR¹⁰, -NR¹⁰R¹¹, -CN, -COR¹⁰ or -COOR¹⁰.

In another aspect, a (non-claimed) method for preparing a second compound is disclosed that interacts with an opioid receptor when a first compound that interacts with an opioid receptor is known, said first compound containing a phenolic hydroxyl, said method comprising converting said phenolic hydroxyl to a residue of formula:

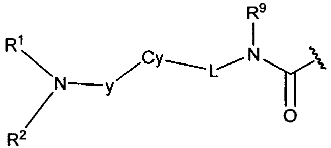

In some embodiments, the residue 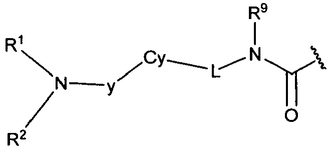 is 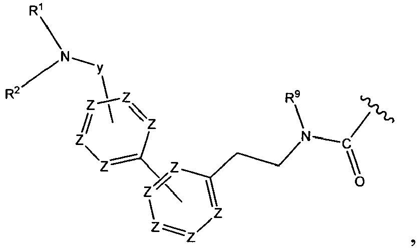 wherein Z is CR¹⁰ or N, with the proviso that, at the points of attachment of the NR¹R²y group to the distal aromatic ring and of the distal aromatic ring to the proximal aromatic ring, Z must be C.

In another aspect, the invention relates to a pharmaceutical formulation comprising a compound of formula I and a pharmaceutically acceptable carrier.

In another aspect, the invention relates to the use in preventing or treating a condition or disease associated with binding opioid receptors in a patient in need thereof, comprising the step of administering to said patient a composition comprising an effective amount of a compound of formula I. In some embodiments, the disease or condition to be treated or prevented is pain, pruritis, diarrhea, irritable bowel syndrome, gastrointestinal motility disorder, obesity, respiratory depression, convulsions, coughing, hyperalgesia and drug addiction. In further embodiments, drug addiction encompasses heroin, cocaine, nicotine or alcohol addiction. In other embodiments, the condition is pain and the composition further comprises an effective amount of an opioid.

### Detailed Description of the Invention

From many years of SAR studies, it is known that the hydroxyl of morphinans and benzomorphans interacts with a specific site in the opiate receptor. We have now surprisingly found that the hydroxyl can be replaced with a very large carboxamide residue. A fairly wide range of secondary carboxamides exhibits binding in the desired range below 25 nanomolar.

Since phenolic hydroxyls of benzomorphans and morphinans can be chemically converted to carboxamides by a simple, flexible and convenient route described in US patents 6,784,187 and 7,057,035, the door is opened to a whole family of new therapeutic agents, many of which derive directly from the application of the principles set forth herein to known therapeutic agents that rely on opioid binding for their activity. Moreover, since the receptor seems to tolerate some variation in Q, one may contemplate further modulating receptor specificity, affinity and tissue distribution by varying the properties of the aryl substituents.

In one aspect the invention relates to compounds of formula I: 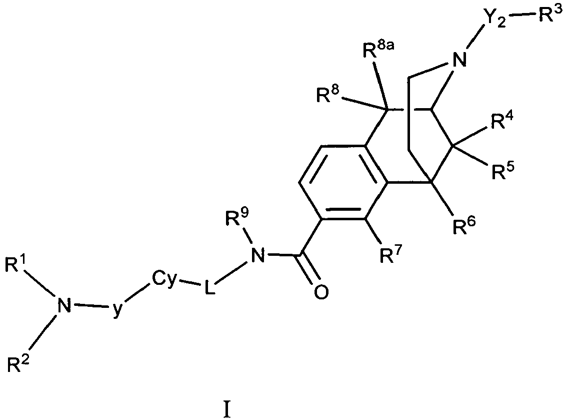 wherein
- R¹ and R²: are each independently chosen from hydrogen, optionally substituted lower alkyl,
- R³: is chosen from hydrogen, C₁-C₈ hydrocarbon, heterocyclyl, aryl and hydroxyalkyl;
- R⁴: is chosen from hydrogen, hydroxyl, C₁-C₂₀ alkyl and C₁-C₂₀ alkyl substituted with hydroxyl or carbonyl;
- R⁵: is lower alkyl;
- R⁶: is lower alkyl;
- R⁷: is chosen from hydrogen, NR¹⁰R¹¹ and -OR¹⁰; or together R⁴, R⁵, R⁶ and R⁷ may form from one to three rings, said rings having optional additional substitution;
- R⁸ and R^{8a}: are both hydrogen or taken together R⁸ and R^{8a} are =O;
- R⁹: is chosen from hydrogen and lower alkyl;
- R¹⁰ and R¹¹: are each independently hydrogen, optionally substituted lower alkyl, optionally substituted alkenyl, optionally substituted alkynyl, optionally substituted aryl, hydroxyl, amino or optionally substituted lower alkoxy;
- y: is -(C(R¹⁰)(R¹¹))p- or a direct bond, wherein p is 0, 1, 2, 3,4, 5, 6, or 7;
- Y₂: is a direct bond or -(C(R¹⁰)(R¹¹))q-, wherein q is 0, 1, 2, 3,4 or 5;
- L: is a direct bond or -(C(R¹⁰)(R¹¹))q-; and
- Cy: is Ar¹-B-Ar², wherein
Ar¹ is absent, or an aryl or heteroaryl radical having from 1 to 4 N, O and/or S atoms, which may be unsubstituted or mono-, di- or trisubstituted by halogen, lower alkyl, alkenyl, alkynyl, cycloalkyl, -OR¹⁰, -NR¹⁰R¹¹, -CN, -COR¹⁰ or - COOR¹⁰;
B is a direct bond, -O-, -NR¹⁰, -SO₂, or -(C(R¹⁰)(R¹¹))s-, wherein s is 0, 1, 2, 3, 4 or 5; and
Ar² is aryl or heteroaryl radical having from 1 to 4 N, O and/or S atoms, which may be unsubstituted or mono-, di- or trisubstituted by halogen, lower alkyl, alkenyl, alkynyl, cycloalkyl, -OR¹⁰, -NR¹⁰R¹¹, -CN, -COR¹⁰ or -COOR¹⁰.

Subclasses of the foregoing structure include:
II. 2,6-methano-3-benzazocines of the structure shown above, in which R⁴, R⁵, R⁶ and R⁷ do not form additional rings: 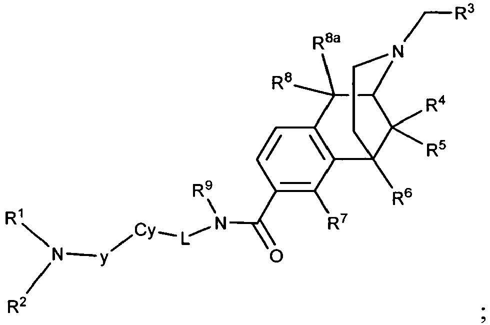
III. morphinans in which R⁵ and R⁶ form one ring: 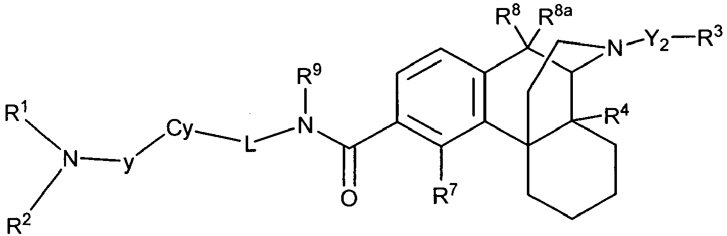 wherein preferably:
   R⁸ and R^{8a} are hydrogen;
   R³ is chosen from hydrogen, cyclopropyl, cyclobutyl, vinyl and tetrahydrofuranyl; and
   R⁴ is hydrogen or hydroxyl;
IV. morphinans in which R⁵, R⁶ and R⁷ form two rings: 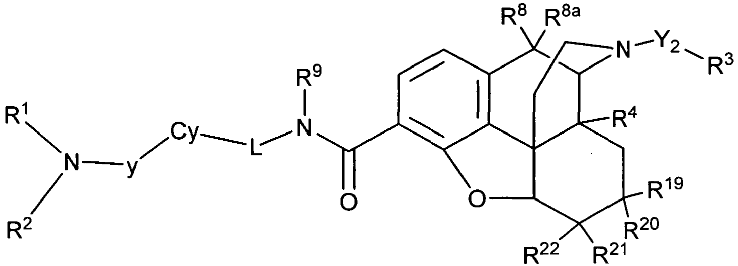 wherein
   - R⁴: is hydrogen or hydroxyl
   - R¹⁹: is hydrogen or lower alkyl;
   - R²⁰: is chosen from hydrogen, lower alkyl and hydroxy(lower alkyl); or together, R¹⁹ and R²⁰ form a spiro-fused carbocycle of 5 to 10 carbons;
   - R²¹: is hydrogen;
   - R²²: is chosen from hydroxyl, lower alkoxy and -NR¹³R¹⁴; or together, R²¹ and R²² form a carbonyl or a vinyl substituent;
   - R¹³: is hydrogen or optionally substituted lower alkoxy; and
   - R¹⁴: is hydrogen, optionally substituted lower alkoxy, acyl or fumarate.
V. morphinans wherein R⁴ and R²¹ form an additional sixth ring, which may be saturated: 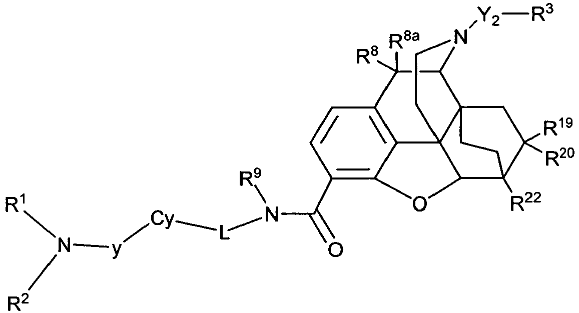 or unsaturated: 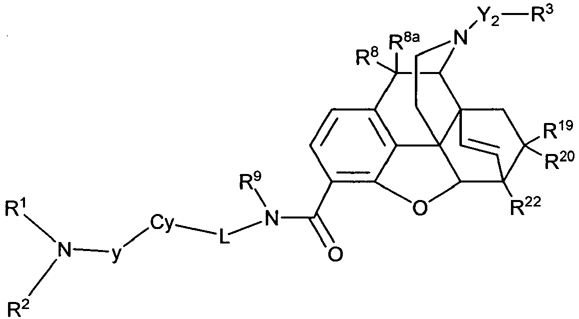 wherein
   - R¹⁹: is hydrogen;
   - R²⁰: is hydroxy(lower alkyl); and
   - R²²: is lower alkoxy.

In some embodiments, an example of Cy includes:

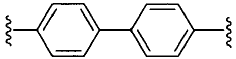

In some embodiments, the invention relates to compounds of formula II:

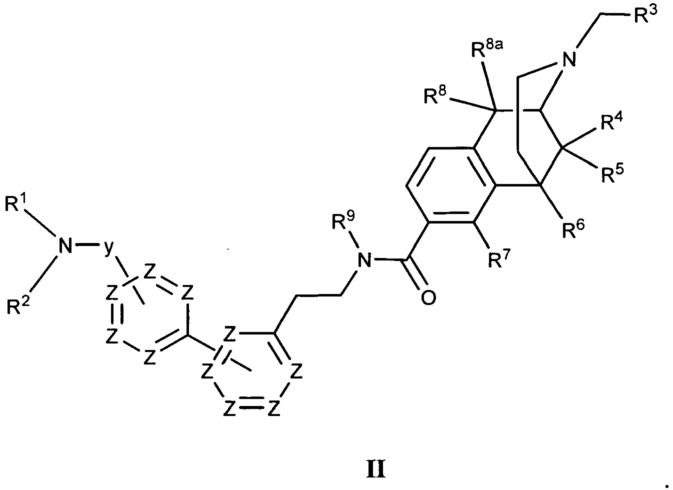

In some embodiments, Z is N. In still other embodiments, Z is CR¹⁰. In further embodiments, R¹⁰ is hydrogen. In other embodiments, R¹⁰ is optionally substituted lower alkyl or optionally substituted lower alkoxy. In further embodiments, R¹⁰ is methyl.

In some embodiments, R¹ and R² are each hydrogen. In other embodiments, R¹ is hydrogen and R² is optionally substituted lower alkyl. In still other embodiments, R¹ and R² are each optionally substituted lower alkyl. In some of these embodiments, R¹ and R² are each methyl. In yet other non-claimed embodiments, R¹ is hydrogen, R² is -COR¹⁰, and R¹⁰, is optionally substituted lower alkoxy. In some of these embodiments, R¹⁰ is tert-butoxy. In still other non-claimed embodiments, R² is, together with the nitrogen to which it is attached, fluorenylmethyl carbamate, tert-butyl carbamate, benzyl carbamate, acetamide, trifluoroacetamide, benzylamine, triphenylmethylamine or toluenesulfonamide. In further non-claimed embodiments, R¹ and R² may form, together with the nitrogen to which they are attached, from one to three rings, said rings having optional additional substitution.

In some embodiments, R³ is hydrogen. In other embodiments, R³ is heterocyclyl. In still other embodiments, R³ is hydroxyalkyl. In yet other embodiments, R³ is C₁-C₈ hydrocarbon. In further embodiments, R³ is cyclopropyl or cyclobutyl.

In some embodiments, R⁴ is hydrogen. In other embodiments, R⁴ is hydroxyl. In non-claimed embodiments, R⁴ is amino or lower alkoxy. In yet other embodiments, R⁴ is C₁-C₂₀ alkyl or C₁-C₂₀ alkyl substituted with hydroxyl or carbonyl. In further embodiments, R⁴ is methyl or ethyl.
- R⁵: is lower alkyl. In some embodiments, R⁵ is methyl.
- R⁶: is lower alkyl. In some embodiments, R⁶ is methyl.

In some embodiments, R⁷ is hydrogen. In other embodiments, R⁷ is -OR¹⁰. In further embodiments, R⁷ is hydroxyl. In still other embodiments, R⁷ is NR¹⁰R¹¹. In further embodiments, R⁷ is NH₂, NHCH₃ or NH(CH₃)₂.

In some embodiments, R⁴, R⁵, R⁶ and R⁷ together may form from one to three rings, said rings having optional additional substitution. Some representative examples are shown above in subgenera III, IV and V.

In an embodiment of the invention, R⁸ and R^{8a} are both hydrogen. In another embodiment, R⁸ and R^{8a} are taken together to form =O.

In some embodiments, R⁹ is hydrogen. In other embodiments, R⁹ is lower alkyl.

In some embodiments, R¹⁰ and R¹¹ are each independently hydrogen. In other embodiments, R¹⁰ is optionally substituted lower alkoxy and R¹¹ is hydrogen or methyl. In still other embodiments, R¹⁰ is optionally substituted lower alkyl and R¹¹ is hydrogen or methyl. In yet other embodiments, R¹⁰ is optionally substituted aryl and R¹¹ is hydrogen or methyl. In yet other embodiments, R¹⁰ is hydroxyl or amino and R¹¹ is hydrogen or methyl.
In an embodiment of the invention, y is CH₂. In another embodiment, y is a direct bond.

In some embodiments, Z is CH. In other embodiments, Z is N. At the points of attachment of the NR¹R²y group to the distal aromatic ring and of the distal aromatic ring to the proximal aromatic ring, Z must be C.

In some embodiments of the invention, formula II has the orientation below:

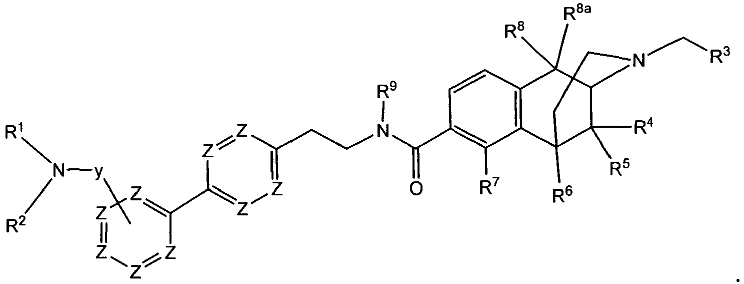

The residue shown here 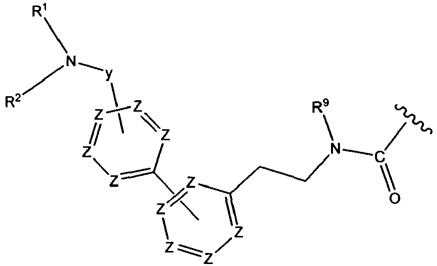 will hereinafter be sometimes referred to as Q.

In certain embodiments of the invention, the aromatic rings of Q have a para orientation:

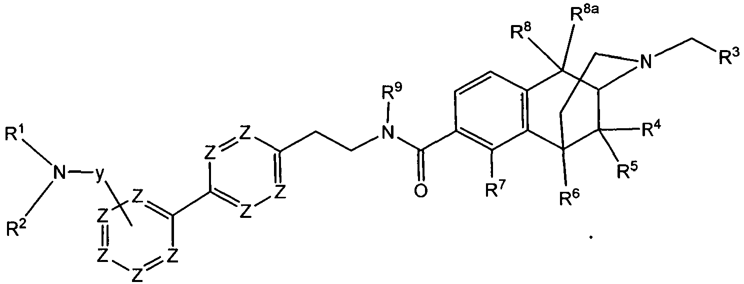

In still other embodiments, each Z is equal to carbon

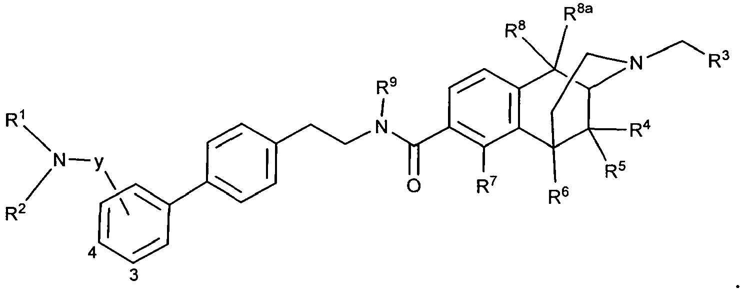

In certain embodiments of formula I and formula II, R⁸, R^{8a} and R⁹ are each hydrogen, R⁵ is methyl and R⁶ is methyl or ethyl. In some of these embodiments, R⁴ is hydrogen. In other embodiments, R⁴ is 3-oxo-5-cyclopentyl-1-pentanyl. In some of these embodiments, R³ is cyclopropyl. In other embodiments, R³ is hydroxycyclopropyl. In other embodiments, R³ is cyclobutyl. In still other embodiments, R³ is hydrogen. In yet other embodiments, R³ is phenyl, furanyl or tetrahydrofuranyl. In further embodiments, R³ is vinyl or dimethylvinyl.

In some embodiments of the invention, -yNR¹R² is attached in the para orientation (the 4-position):

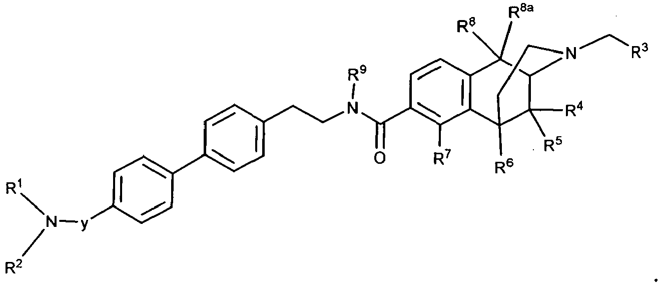

In some of these embodiments, y is a direct bond. In yet other embodiments, R¹ and R² are each equal to lower alkyl. In some embodiments, R¹ and R² are each selected from hydrogen and methyl. In further embodiments, R¹ and R² are both methyl. In other embodiments, R¹ is hydrogen and R² is substituted lower alkyl. For instance, R² could be triphenylmethyl or benzyl. In non-claimed embodiments, R¹ is hydrogen and R² is -SO₂R¹⁰. In some of these embodiments, R¹⁰ is optionally substituted aryl, for instance, toluene. In still other non-claimed embodiments, R¹ is hydrogen and R² is -COR¹⁰. In some of these embodiments, R¹⁰ is optionally substituted lower alkoxy, for instance, fluorenylmethoxy, t-butoxy, or benzyloxy. In other of these embodiments, R¹⁰ is optionally substituted lower alkyl, for instance, methyl or trifluoromethyl. In some of the non-claimed embodiments, R² is, together with the nitrogen to which it is attached, fluorenylmethyl carbamate, tert-butyl carbamate, benzyl carbamate, acetamide, trifluoroacetamide, benzylamine, triphenylmethylamine or toluenesulfonamide. In still other non-claimed embodiments, -NR¹R² together form from one to three optionally substituted rings. One example is phthalimide.

In some of these embodiments, y is -CH₂. In yet other embodiments, R¹ and R² are each equal to lower alkyl. In some embodiments, R¹ and R² are each selected from hydrogen and methyl. In further embodiments, R¹ and R² are both methyl. In other embodiments, R¹ is hydrogen and R² is substituted lower alkyl. For instance, R² could be triphenylmethyl or benzyl. In non-claimed embodiments, R¹ is hydrogen and R² is -SO₂R¹⁰. In some of these embodiments, R¹⁰ is optionally substituted aryl, for instance, toluene. In still other non-claimed embodiments, R¹ is hydrogen and R² is -COR¹⁰. In some of these embodiments, R¹⁰ is optionally substituted lower alkoxy, for instance, fluorenylmethoxy, t-butoxy, or benzyloxy. In other of these embodiments, R¹⁰ is optionally substituted lower alkyl, for instance, methyl or trifluoromethyl. In some of the non-claimed embodiments, R² is, together with the nitrogen to which it is attached, fluorenylmethyl carbamate, tert-butyl carbamate, benzyl carbamate, acetamide, trifluoroacetamide, benzylamine, triphenylmethylamine or toluenesulfonamide. In still other non-claimed embodiments, -NR¹R² together form from one to three optionally substituted rings. One example is phthalimide.

In some embodiments of the invention, -yNR¹R² is attached in the meta orientation (the 3-position). In some of these embodiments, y is a direct bond. In other embodiments, R¹ and R² are each selected from hydrogen and methyl. In other embodiments, R¹ is hydrogen and R² is substituted lower alkyl. For instance, R² could be triphenylmethyl or benzyl. In non-claimed embodiments, R¹ is hydrogen and R² is -SO₂R¹⁰. In some of these embodiments, R¹⁰ is optionally substituted aryl, for instance, toluene. In still other non-claimed embodiments, R¹ is hydrogen and R² is -COR¹⁰. In some of these embodiments, R¹⁰ is optionally substituted lower alkoxy, for instance, fluorenylmethoxy, t-butoxy, or benzyloxy. In other of these embodiments, R¹⁰ is optionally substituted lower alkyl, for instance, methyl or trifluoromethyl. In some of the non-claimed embodiments, R² is, together with the nitrogen to which it is attached, fluorenylmethyl carbamate, tert-butyl carbamate, benzyl carbamate, acetamide, trifluoroacetamide, benzylamine, triphenylmethylamine or toluenesulfonamide. In still other non-claimed embodiments, -NR¹R² together form from one to three optionally substituted rings. One example is phthalimide.

In some embodiments, R⁵ and R⁶ together form one ring:

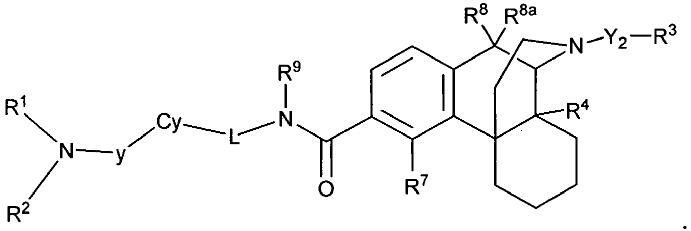

In some of these embodiments, R⁴, R⁸ and R^{8a} are each hydrogen. In other embodiments, R⁸ and R^{8a} are each hydrogen and R⁴ is hydroxyl. In non-claimed embodiments, R⁴ is amino. In some of these embodiments, R³ is hydrogen. In other embodiments, R³ is cyclopropyl or cyclobutyl. In still other embodiments, R³ is vinyl. In yet other embodiments, R³ is tetrahydrofuranyl. In some embodiments, the compounds are of formula

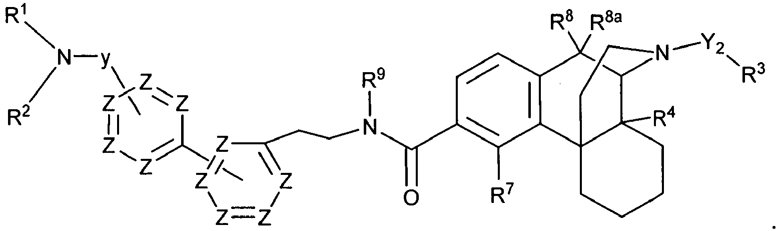

In some embodiments, together R⁵, R⁶ and R⁷ form two rings, having the structure:

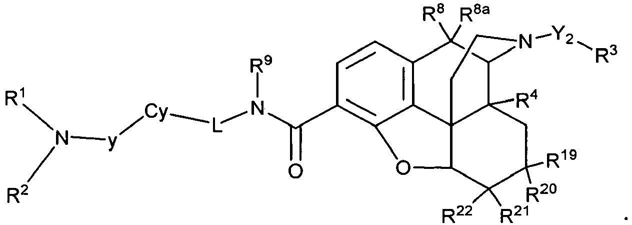

In these embodiments, R¹⁹ is hydrogen or lower alkyl; and R²¹ is hydrogen. In some of these embodiments, R²⁰ is chosen from hydrogen, lower alkyl and hydroxy(lower alkyl) In other embodiments, R¹⁹ and R²⁰ together form a spiro-fused carbocycle of 5 to 10 carbons. In yet other embodiments, R²² is chosen from hydroxy, lower alkoxy and -NR¹³R¹⁴. In still other embodiments, R¹³ is hydrogen or optionally substituted lower alkoxy. In yet other embodiments, R¹⁴ is hydrogen, optionally substituted lower alkoxy, acyl or fumarate.

In still other embodiments, R²¹ and R²² together form a carbonyl or a vinyl substituent. In some embodiments, (a) the compounds are of formula

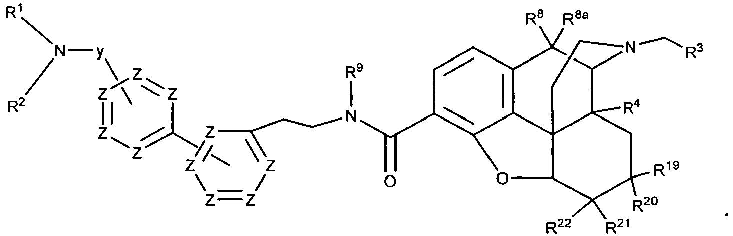

In other embodiments (b), together, R⁴ and R²¹ form a sixth ring exemplified below:

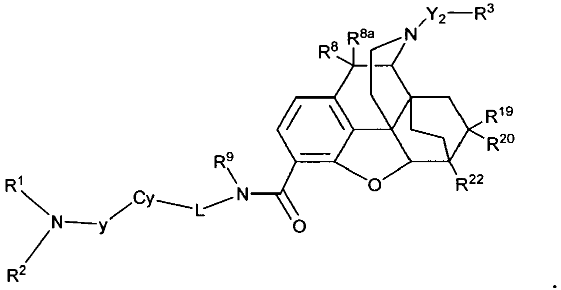

In some of these embodiments (c), the compounds are of formula

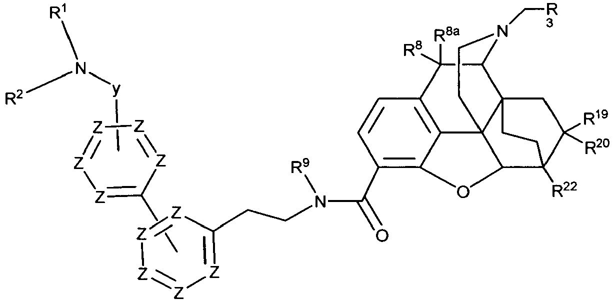

In another embodiment (d), R⁴ and R²¹ form a sixth ring exemplified by:

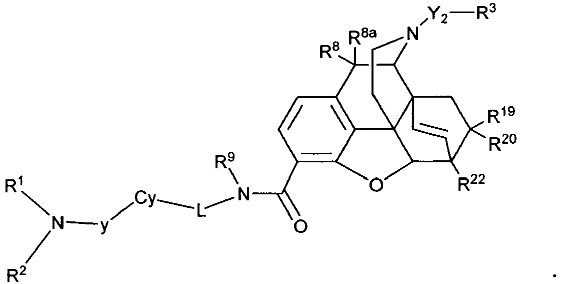

In some embodiments (e), the compounds are of formula

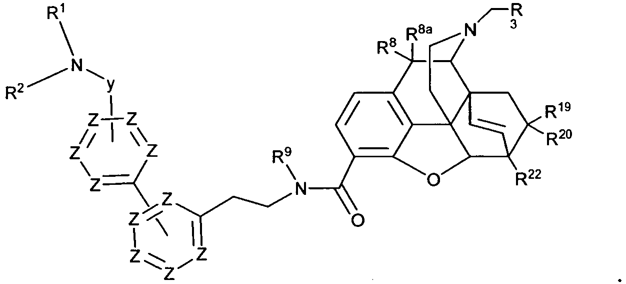

In the embodiments (b), (c), (d) and (e), R¹⁹ is hydrogen; R²⁰ is hydroxy(lower alkyl); and R²² is lower alkoxy.

In a non-claimed aspect, a method for preparing a second compound is provided that interacts with an opioid receptor when a first compound that interacts with an opioid receptor is known. When the first compound contains a phenolic hydroxyl, the method comprises converting the phenolic hydroxyl to a residue of structure:

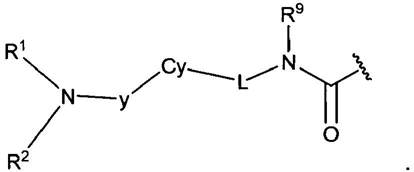

In some embodiments, the residue is 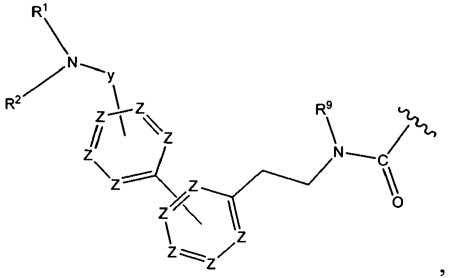 which will be sometimes referred to as Q.

It is known in the art that compounds that are µ, δ and κ agonists exhibit analgesic activity; compounds that are selective µ agonists exhibit anti-diarrheal activity and are useful in treating dyskinesia; µ antagonists and κ agonists are useful in treating heroin, cocaine, alcohol and nicotine addiction; κ agonists are also anti-pruritic agents and are useful in treating hyperalgesia. Recently it has been found [Peterson et al. Biochem. Pharmacol. 61, 1141-1151 (2001)] that κ agonists are also useful in treating retroviral infections. In general, the dextrorotatory isomers of morphinans of type III above are useful as antitussives and anticonvulsants.

Opioid receptor ligands having known high affinity are shown in the following charts. Replacement of OH with the 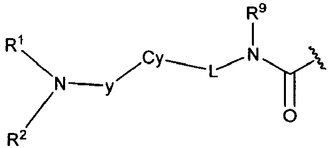 residue or with Q in these compounds produces compounds that exhibit similar activity and better bioavailability.

### Chart 1. Opioid Receptor Ligands Benzomorphinans (a.k.a. 2,6-Methano-3-benzazocines)

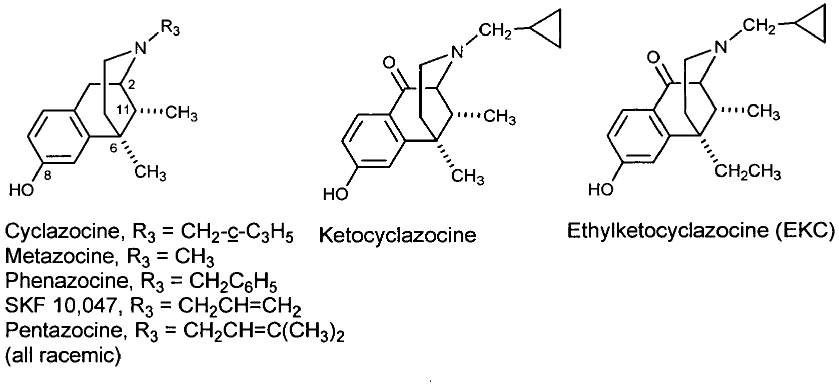

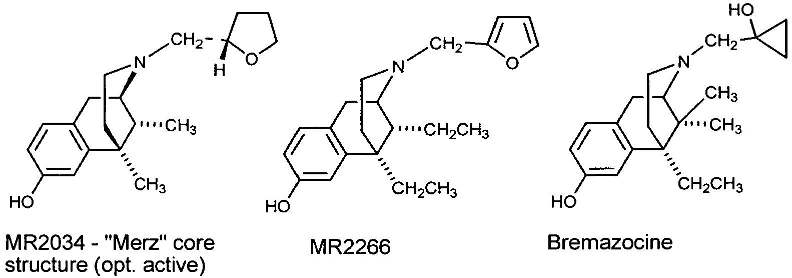

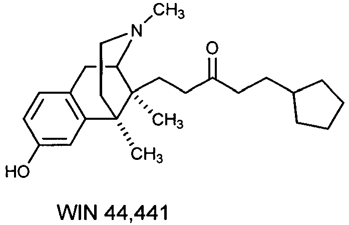

### Chart 2. Opioid Receptor Ligands Morphine and Morphinans

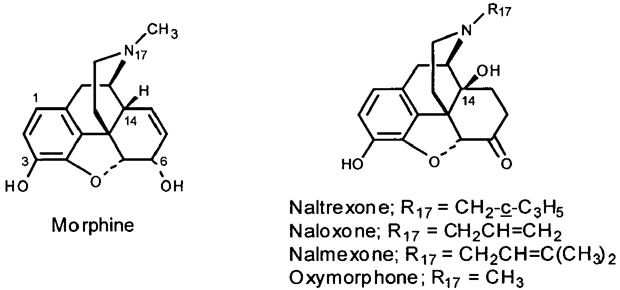

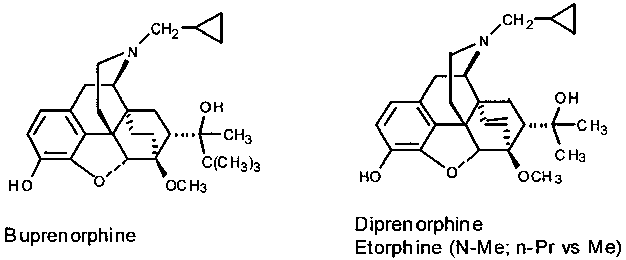

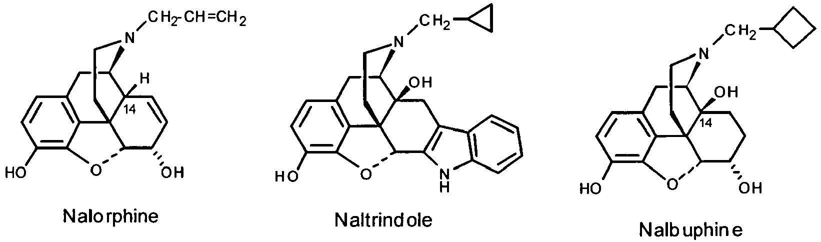

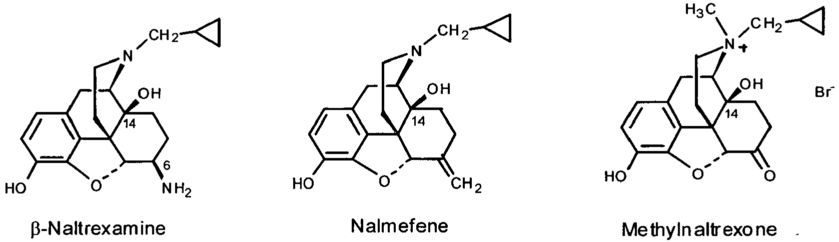

### Chart 2 (continued). Opioid Receptor Ligands Morphine and Morphinans

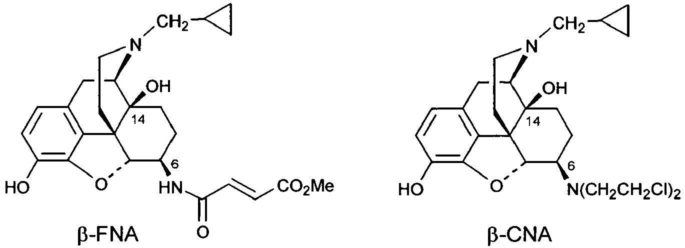

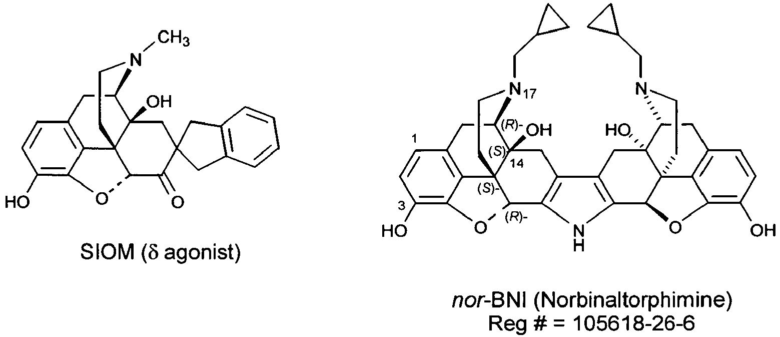

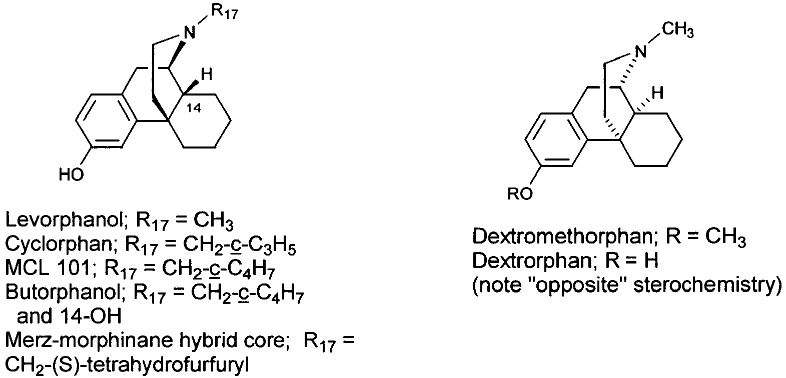

### Chart 3 - Miscellaneous Opioid Receptor Ligands

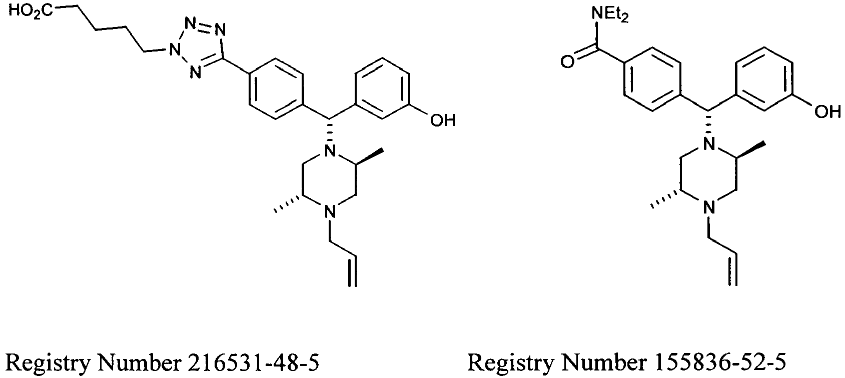

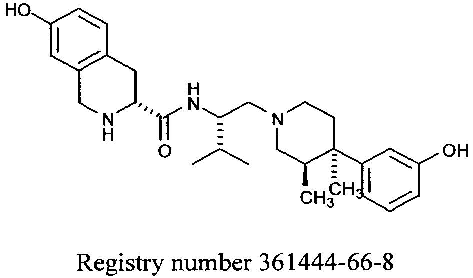

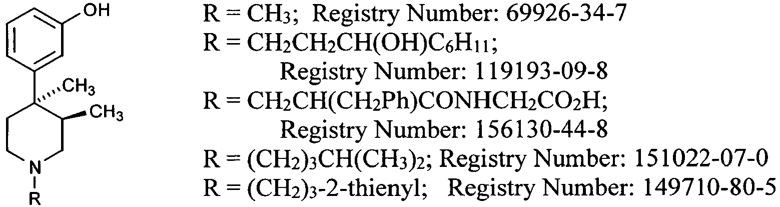

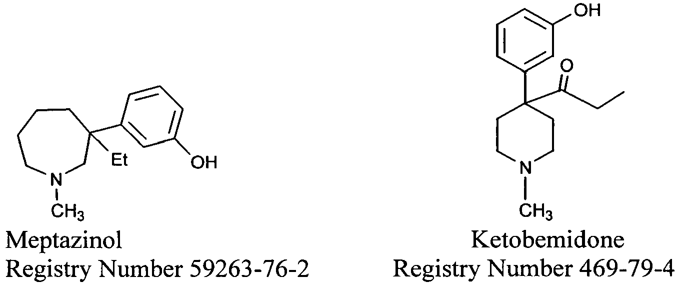

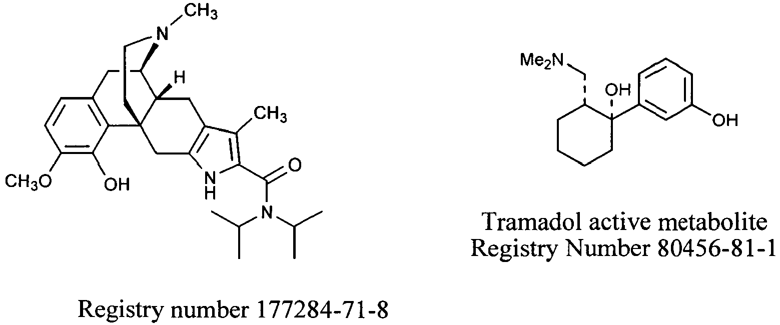

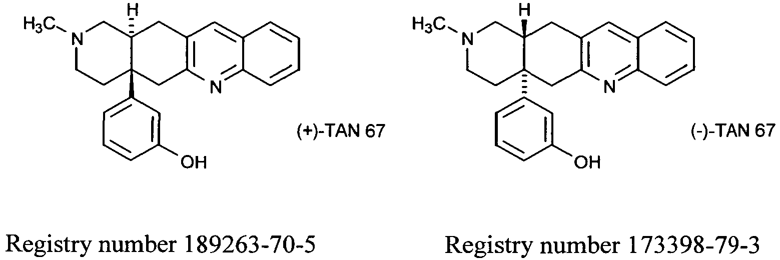

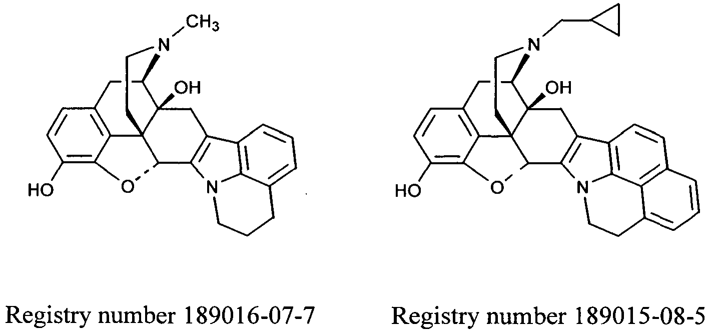

Other opioid receptor ligands are described in Aldrich, J.V. "Analgesics" in Burger's Medicinal Chemistry and Drug Discovery, M.E.Wolff ed., John Wiley & Sons 1996, pages 321-44. In all but two of the foregoing compounds, there is a single phenolic OH that is to be replaced by the 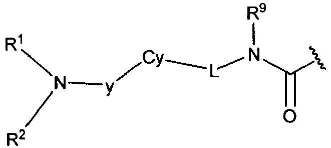 residue or by Q according to the present invention. In norbinaltorphimine and 361444-66-8, there are two phenolic OH's, either or both of which are replaced by the 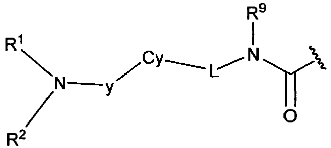 residue or by Q.

Binding assays used to screen compounds are similar to those previously reported by Neumeyer et al., Design and Synthesis of Novel Dimeric Morphinan Ligands for κ and µ Opioid Receptors. J. Med. Chem. 2003, 46, 5162. Membrane protein from CHO cells that stably expressed one type of the human opioid receptor were incubated with 12 different concentrations of the compound in the presence of either 1 nM [³H]U69,593¹⁰ (κ), 0.25 nM [³H]DAMGO¹¹ (µ) or 0.2 nM [³H]naltrindole¹² (δ) in a final volume of 1 mL of 50 mM Tris-HCl, pH 7.5 at 25°C. Incubation times of 60 min were used for [³H]U69,593 and [³H]DAMGO. Because of a slower association of [³H]naltrindole with the receptor, a 3 h incubation was used with this radioligand. Samples incubated with [³H]naltrindole also contained 10 mM MgCl₂ and 0.5 mM phenylmethylsulfonyl fluoride. Nonspecific binding was measured by inclusion of 10 µM naloxone. The binding was terminated by filtering the samples through Schleicher & Schuell No. 32 glass fiber filters using a Brandel 48-well cell harvester. The filters were subsequently washed three times with 3 mL of cold 50 mM Tris-HCl, pH 7.5, and were counted in 2 mL Ecoscint A scintillation fluid. For [³H]naltrindole and [³H]U69,593 binding, the filters were soaked in 0.1% polyethylenimine for at least 60 min before use. IC₅₀ values were-calculated by least squares fit to a logarithm-probit analysis. *Kᵢ* values of unlabeled compounds were calculated from the equation *Kᵢ* = (IC₅₀)/1+S where S = (concentration of radioligand)/(*K*_{d} of radioligand).¹³ Data are the mean ± SEM from at least three experiments performed in triplicate.

[³⁵S]GTPγS Binding Assays. In a final volume of 0.5 mL, 12 different concentrations of each test compound were incubated with 15 µg (κ), 10 µg (δ) or 7.5 µg (µ) of CHO cell membranes that stably expressed either the human κ, δ or µ opioid receptor. The assay buffer consisted of 50 mM Tris-HCl, pH 7.4, 3 mM MgCl₂, 0.2 mM EGTA, 3 µM GDP, and 100 mM NaCl. The final concentration of [³⁵S]GTPγS was 0.080 nM. Nonspecific binding was measured by inclusion of 10 µM GTPγS. Binding was initiated by the addition of the membranes. After an incubation of 60 min at 30°C, the samples were filtered through Schleicher & Schuell No. 32 glass fiber filters. The filters were washed three times with cold 50 mM Tris-HCl, pH 7.5, and were counted in 2 mL of Ecoscint scintillation fluid. Data are the mean Eₘₐₓ and EC₅₀ values ± S.E.M. from at least three separate experiments, performed in triplicate. For calculation of the Eₘₐₓ values, the basal [³⁵S]GTPγS binding was set at 0%. To determine antagonist activity of a compound at the µ opioid receptors, CHO membranes expressing the µ opioid receptor, were incubated with 12 different concentrations of the compound in the presence of 200 nM of the µ agonist DAMGO. To determine antagonist activity of a compound at the κ opioid receptors, CHO membranes expressing the κ opioid receptor, were incubated with the compound in the presence of 100 nM of the κ agonist U50,488. To determine if a compound was an antagonist at δ receptors, CHO membranes expressing the δ receptor were incubated with 12 different concentrations of the test compound in the presence of 10 nM of the δ-selective agonist SNC 80.

### Examples - Cyclazocine subseries

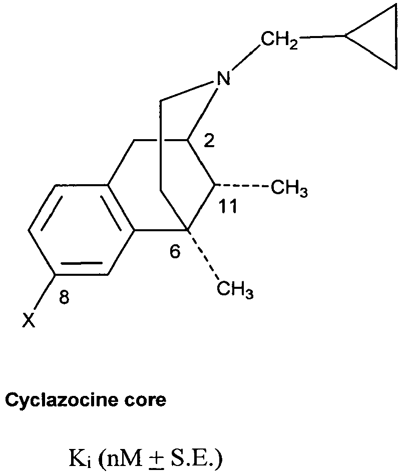

| Example No. | X | [³H]DAMGO (µ) | *K*ᵢ (nM) [³H]Naltrindole (δ) | [³H]U69,593 (κ) |
|---|---|---|---|---|
| MV-E-126 | CONH(CH₂)₂(4-C₆H₄-4-(CH₃)₂NC₆H₄) | 0.087 ± 0.0077 | 1.4 ± 0.071 | 0.76 ± 0.12 |
| SJJ-B-074c | CONH(CH₂)₂(4-C₆H₄-3-(CH₃)₂NC₆H₄) | 0.18 ± 0.055 | 2.5 ± 0.17 | 0.26 ± 0.022 |
| SJJ-B-112g | CONH(CH₂)₂(4-C₆H₄-4-NH₂C₆H₄) | 0.0014 ± 0.00010 | 1.5 ± 0.078 | 0.39 ± 0.0085 |
| SJJ-C-027b | CONH(CH₂)₂(4-C₆H₄-4-BocNHC₆H₄) | 0.32 ± 0.015 | 3.1 ± 0.34 | 3.4 ± 0.32 |
| SJJ-C-013b | CONH(CH₂)₂(4-C₆H₄-4-(CH₃)₂NCH₂C₆H₄) | 0.094 ± 0.0054 | 3.7 ± 0.15 | 1.9 ± 0.014 |

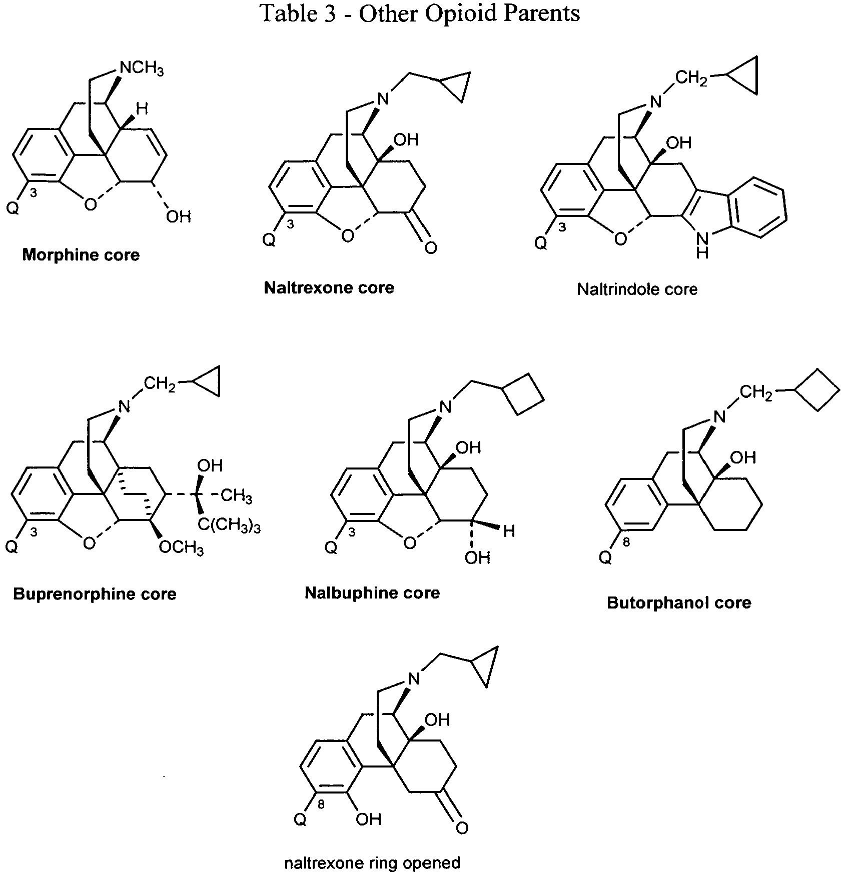

Antinociceptive activity is evaluated by the method described in Jiang et al. [J. Pharmacol. Exp. Ther. 264, 1021-1027 (1993), page 1022]. The ED_{50'S} of compounds of the invention are expected to be under 100 nmol in the mouse acetic acid writhing test when administered i.c.v., and an increase in the duration of action is expected for compounds of the invention compared to their "parents" when given by i.p. administration.

### Definitions

Throughout this specification the terms and substituents retain their definitions.

Alkyl is intended to include linear, branched, or cyclic hydrocarbon structures and combinations thereof. A combination would be, for example, cyclopropylmethyl. Lower alkyl refers to alkyl groups of from 1 to 6 carbon atoms. Examples of lower alkyl groups include methyl, ethyl, propyl, isopropyl, cyclopropyl, butyl, s-and t-butyl, cyclobutyl and the like. Preferred alkyl groups are those of C₂₀ or below. Cycloalkyl is a subset of alkyl and includes cyclic hydrocarbon groups of from 3 to 8 carbon atoms. Examples of cycloalkyl groups include c-propyl, c-butyl, c-pentyl, norbornyl and the like.

Alkoxy or alkoxyl refers to groups of from 1 to 8 carbon atoms of a straight, branched, or cyclic configuration and combinations thereof attached to the parent structure through an oxygen. Examples include methoxy, ethoxy, propoxy, isopropoxy, cyclopropyloxy, cyclohexyloxy and the like. Lower-alkoxy refers to groups containing one to four carbons.

Aryl and heteroaryl mean a 5- or 6-membered aromatic or heteroaromatic ring containing 0-3 heteroatoms selected from O, N, or S; a bicyclic 9- or 10-membered aromatic or heteroaromatic ring system containing 0-3 heteroatoms selected from O, N, or S; or a tricyclic 13- or 14-membered aromatic or heteroaromatic ring system containing 0-3 heteroatoms selected from O, N, or S. The aromatic 6- to 14-membered carbocyclic rings include, *e.g*., benzene, naphthalene, indane, tetralin, and fluorene and the 5- to 10-membered aromatic heterocyclic rings include, *e.g*., imidazole, pyridine, indole, thiophene, benzopyranone, thiazole, furan, benzimidazole, quinoline, isoquinoline, quinoxaline, pyrimidine, pyrazine, tetrazole and pyrazole. As used herein aryl and heteroaryl refer to residues in which one or more rings are aromatic, but not all need be.

Arylalkyl means an alkyl residue attached to an aryl ring. Examples are benzyl, phenethyl and the like. Heteroarylalkyl means an alkyl residue attached to a heteroaryl ring. Examples include, e.g., pyridinylmethyl, pyrimidinylethyl and the like.

C₁ to C₂₀ hydrocarbon means a linear, branched, or cyclic residue comprised of hydrogen and carbon as the only elemental constituents and includes alkyl, cycloalkyl, polycycloalkyl, alkenyl, alkynyl, aryl and combinations thereof. Examples include benzyl, phenethyl, cyclohexylmethyl, camphoryl and naphthylethyl.

Heterocycle means a cycloalkyl or aryl residue in which one to two of the carbons is replaced by a heteroatom such as oxygen, nitrogen or sulfur. Heteroaryls form a subset of heterocycles. Examples of heterocycles that fall within the scope of the invention include pyrrolidine, pyrazole, pyrrole, indole, quinoline, isoquinoline, tetrahydroisoquinoline, benzofuran, benzodioxan, benzodioxole (commonly referred to as methylenedioxyphenyl, when occurring as a substituent), tetrazole, morpholine, thiazole, pyridine, pyridazine, pyrimidine, thiophene, furan, oxazole, oxazoline, isoxazole, dioxane, tetrahydrofuran and the like.

Substituted alkyl, aryl, cycloalkyl, heterocyclyl etc. refer to alkyl, aryl, cycloalkyl, or heterocyclyl wherein up to three H atoms in each residue are replaced with halogen, haloalkyl, alkyl, acyl, alkoxyalkyl, hydroxyloweralkyl, phenyl, heteroaryl, benzenesulfonyl, hydroxy, loweralkoxy, haloalkoxy, carboxy, carboalkoxy (also referred to as alkoxycarbonyl), alkoxycarbonylamino, carboxamido (also referred to as alkylaminocarbonyl), cyano, carbonyl, acetoxy, nitro, amino, alkylamino, dialkylamino, mercapto, alkylthio, sulfoxide, sulfone, sulfonylamino, acylamino, amidino, aryl, benzyl, heterocyclyl, phenoxy, benzyloxy, heteroaryloxy, hydroxyimino, alkoxyimino, oxaalkyl, aminosulfonyl, trityl, amidino, guanidino, ureido, and benzyloxy.

Virtually all of the compounds described herein contain one or more asymmetric centers and may thus give rise to enantiomers, diastereomers, and other stereoisomeric forms that may be defined, in terms of absolute stereochemistry, as (R)- or (S)-. The present invention is meant to include all such possible isomers, as well as their racemic and optically pure forms. In general it has been found that the levo isomer of morphinans and benzomorphans is the more potent antinociceptive agent, while the dextro isomer may be useful as an antitussive or antispasmodic agent. Optically active (R)- and (S)- isomers may be prepared using chiral synthons or chiral reagents, or resolved using conventional techniques. When the compounds described herein contain olefinic double bonds or other centers of geometric asymmetry, and unless specified otherwise, it is intended that the compounds include both E and Z geometric isomers. Likewise, all tautomeric forms are also intended to be included.

Some of the compounds of the invention are quaternary salts, i.e. cationic species. Therefore they will always be presented as salts, and the term "pharmaceutically acceptable salt" refers to salts whose counter ion (anion) derives from pharmaceutically acceptable non-toxic acids including inorganic acids, organic acids and water (which formally furnishes the hydroxide anion). Suitable pharmaceutically acceptable anions for the compounds of the present invention include hydroxide, acetate, benzenesulfonate (besylate), benzoate, bicarbonate, bisulfate, carbonate, camphorsulfonate, citrate, ethanesulfonate, fumarate, gluconate, glutamate, glycolate, bromide, chloride, isethionate, lactate, maleate, malate, mandelate, methanesulfonate, mucate, nitrate, pamoate, pantothenate, phosphate, succinate, sulfate, tartrate, trifluoroacetate, p-toluenesulfonate, acetamidobenzoate, adipate, alginate, aminosalicylate, anhydromethylenecitrate, ascorbate, aspartate, calcium edetate, camphorate, camsylate, caprate, caproate, caprylate, cinnamate, cyclamate, dichloroacetate, edetate (EDTA), edisylate, embonate, estolate, esylate, fluoride, formate, gentisate, gluceptate, glucuronate, glycerophosphate, glycolate, glycollylarsanilate, hexylresorcinate, hippurate, hydroxynaphthoate, iodide, lactobionate, malonate, mesylate, napadisylate, napsylate, nicotinate, oleate, orotate, oxalate, oxoglutarate, palmitate, pectinate, pectinate polymer, phenylethylbarbiturate, picrate, pidolate, propionate, rhodanide, salicylate, sebacate, stearate, tannate, theoclate, tosylate and the like. The desired salt may be obtained by ion exchange of whatever counter ion is obtained in the synthesis of the quat. These methods are well known to persons of skill. Although pharmaceutically acceptable counter ions will be preferred for preparing pharmaceutical formulations, other anions are quite acceptable as synthetic intermediates. Thus X may be pharmaceutically undesirable anions, such as iodide, oxalate, trifluoromethanesulfonate and the like, when such salts are chemical intermediates. When the compounds of the invention are bisquats, one may employ as counter ions either two monoanionic species (e.g. Cl₂) or a single dianionic species (e.g. fumarate). Similarly, one could employ oligoanionic species and make salts having appropriate ratios of quat to counterion, such as (quat)₃ citrates. These would be obvious equivalents.

### Abbreviations

The following abbreviations and terms have the indicated meanings throughout:
- Ac: = acetyl
- BNB: = 4-bromomethyl-3-nitrobenzoic acid
- Boc: = t-butyloxy carbonyl
- BPE: = 2(4-biphenylyl)ethyl = 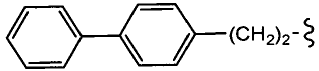
- Bu: = butyl
- c-: = cyclo
- DAMGO: = Tyr-ala-Gly-NMePhe-NHCH₂OH
- DBU: = diazabicyclo[5.4.0]undec-7-ene
- DCM: = dichloromethane = methylene chloride = CH₂Cl₂
- DEAD: = diethyl azodicarboxylate
- DIC: = diisopropylcarbodiimide
- DIEA: = N,N-diisopropylethyl amine
- DMAP: = 4-N,N-dimethylaminopyridine
- DMF: = N,N-dimethylformamide
- DMSO: = dimethyl sulfoxide
- DOR: = delta opioid receptor
- DPPF: = 1,1'-bis(diphenylphosphino)ferrocene
- DVB: = 1,4-divinylbenzene
- EEDQ: = 2-ethoxy-1-ethoxycarbonyl-1,2-dihydroquinoline
- Fmoc: = 9-fluorenylmethoxycarbonyl
- GC: = gas chromatography
- HATU: = O-(7-Azabenzotriazol-1-yl)-1,1,3,3-tetramethyluronium hexafluorophosphate
- HOAc: = acetic acid
- HOBt: = hydroxybenzotriazole
- KOR: = kappa opioid receptor
- Me: = methyl
- mesyl: = methanesulfonyl
- MOR: = mu opioid receptor
- MTBE: = methyl t-butyl ether
- NMO: = N-methylmorpholine oxide
- PEG: = polyethylene glycol
- Ph: = phenyl
- PhOH: = phenol
- PfP: = pentafluorophenol
- PPTS: = pyridinium p-toluenesulfonate
- PyBroP: = bromo-tris-pyrrolidino-phosphonium hexafluorophosphate
- rt: = room temperature
- sat'd: = saturated
- s-: = secondary
- t-: = tertiary
- TBDMS: = t-butyldimethylsilyl
- TFA: = trifluoroacetic acid
- THF: = tetrahydrofuran
- TMOF: = trimethyl orthoformate
- TMS: = trimethylsilyl
- tosyl: = p-toluenesulfonyl
- Trt: = triphenylmethyl
- U69,593: =

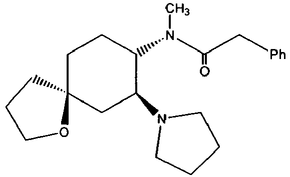

It may happen that residues in the substrate of interest require protection and deprotection during the conversion of the phenol to the desired Q. Terminology related to "protecting", "deprotecting" and "protected" functionalities occurs throughout this application. Such terminology is well understood by persons of skill in the art and is used in the context of processes which involve sequential treatment with a series of reagents. In that context, a protecting group refers to a group which is used to mask a functionality during a process step in which it would otherwise react, but in which reaction is undesirable. The protecting group prevents reaction at that step, but may be subsequently removed to expose the original functionality. The removal or "deprotection" occurs after the completion of the reaction or reactions in which the functionality would interfere. Thus, when a sequence of reagents is specified, as it is below, the person of ordinary skill can readily envision those groups that would be suitable as "protecting groups". Suitable groups for that purpose are discussed in standard textbooks in the field of chemistry, such as Protective Groups in Organic Synthesis by T.W.Greene [John Wiley & Sons, New York, 1991].

The compounds of the invention are synthesized by one of the routes described below:

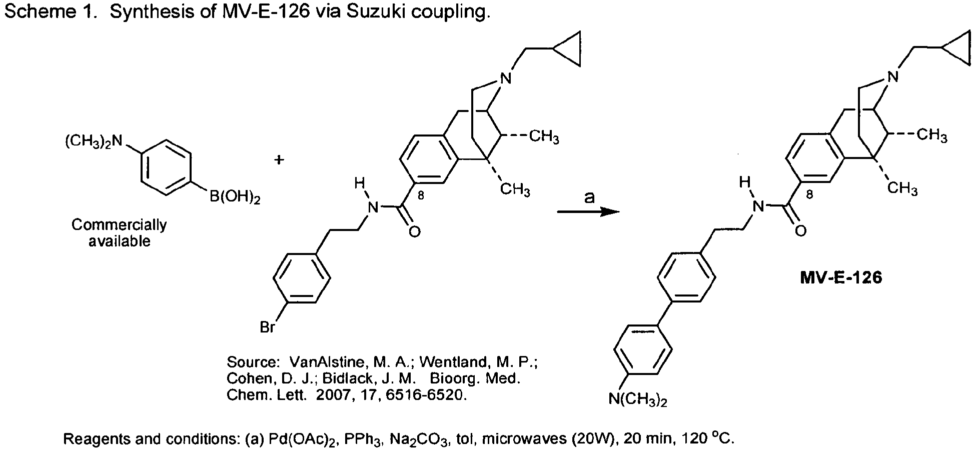

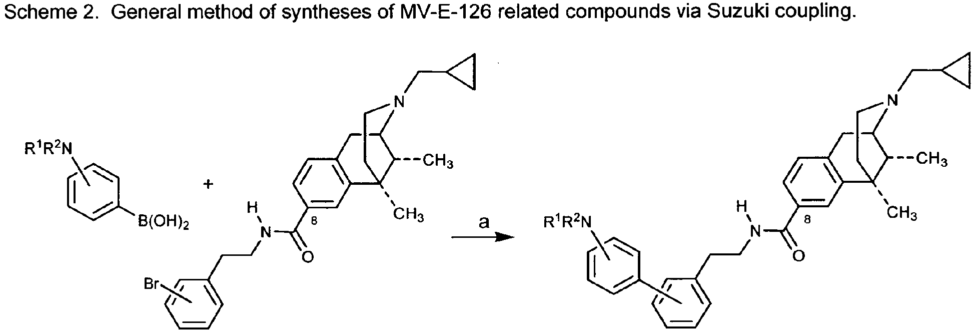

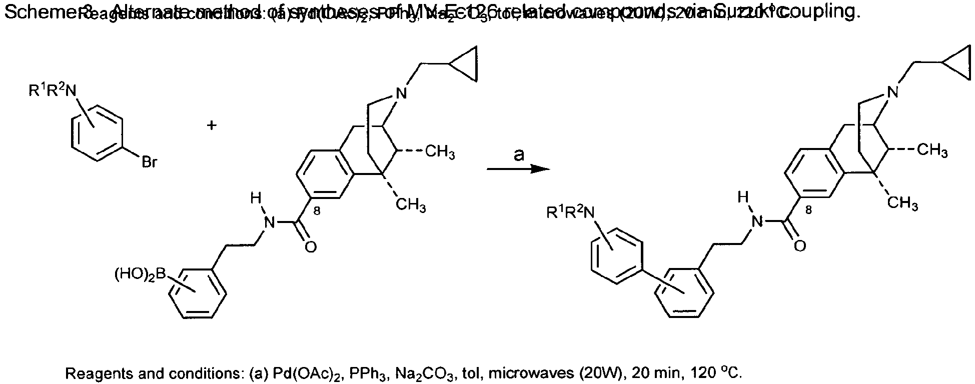

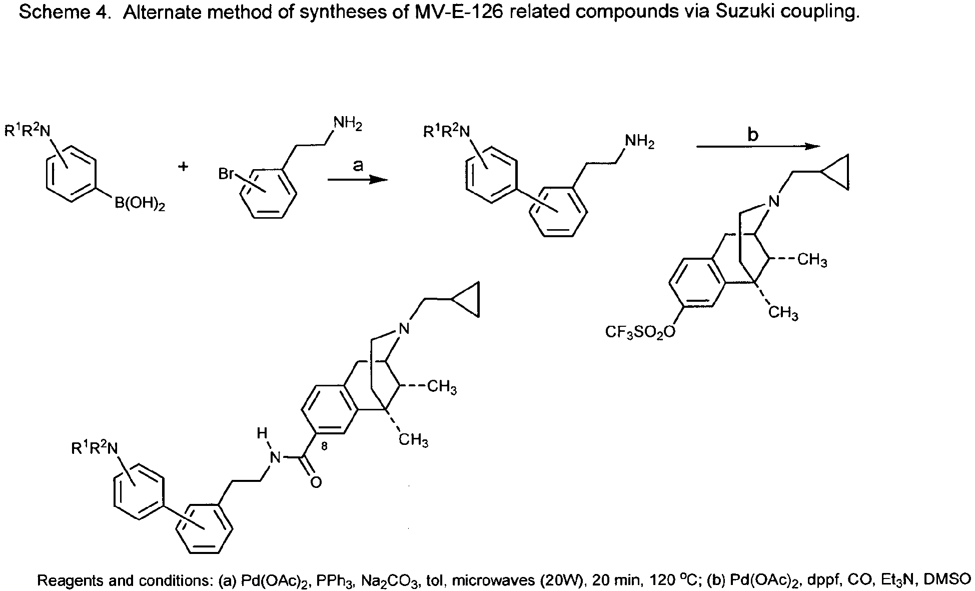

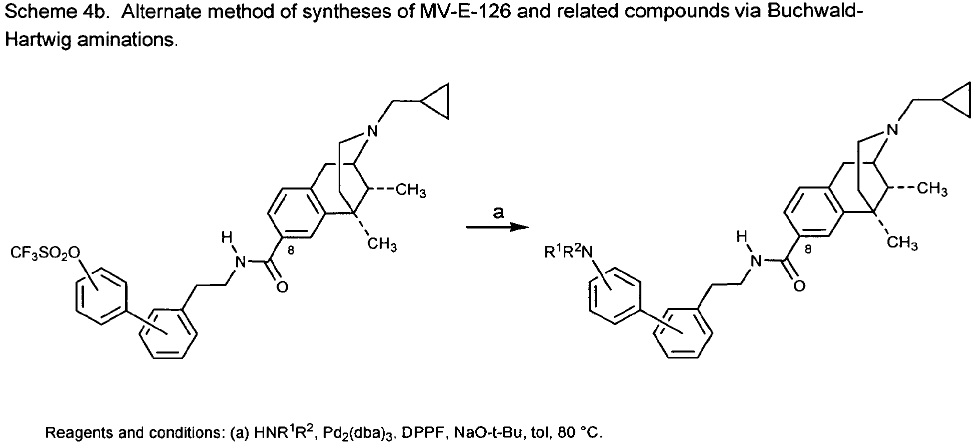

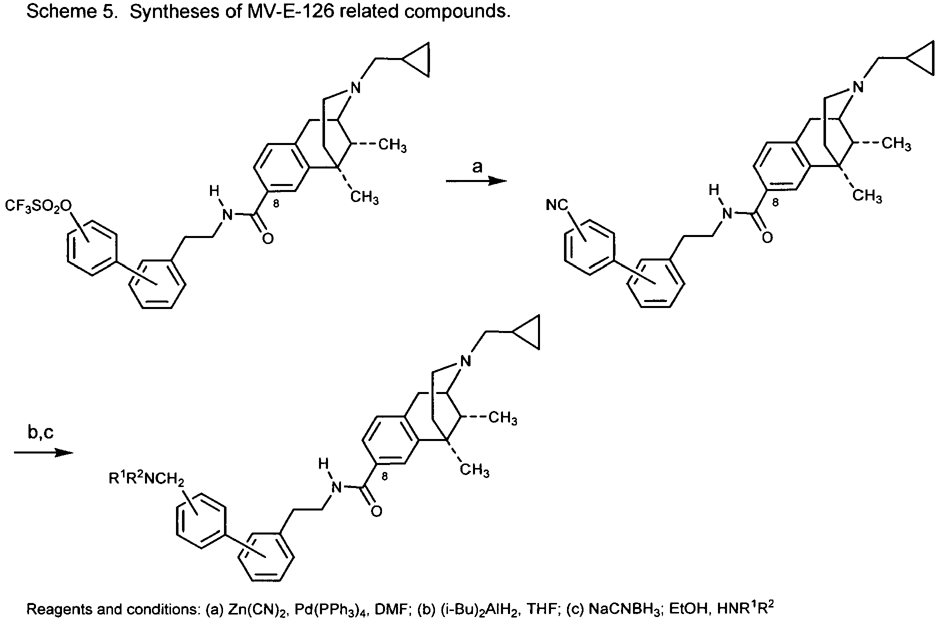

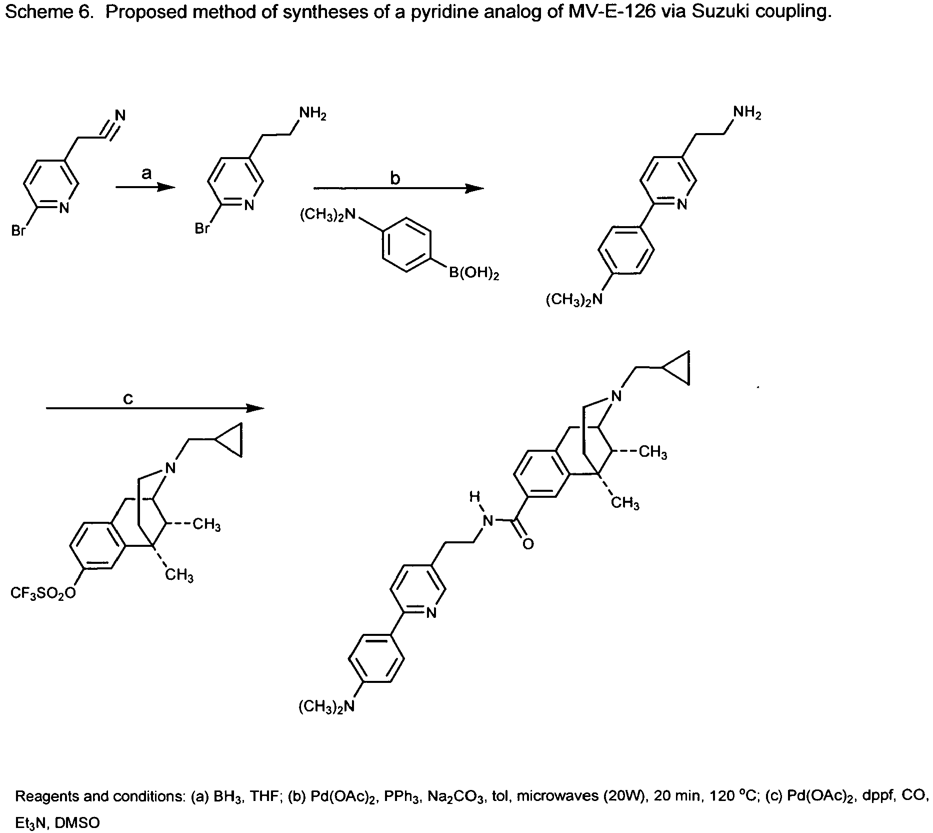

In general, the method of replacing a phenolic -OH with triflate, as shown in Scheme 4, is described in US patent 6,784,187.

Proton NMR spectra and in certain cases ¹³C NMR were obtained on a Varian Unity-300 or 500 NMR spectrometer with tetramethylsilane as an internal reference for samples dissolved in CDCl₃. Samples dissolved in CD₃OD and DMSO-*d*₆ were referenced to the solvent. Proton NMR multiplicity data are denoted by s (singlet), d (doublet), t (triplet), q (quartet), m (multiplet), dd (doublet of doublets), and br (broad). Coupling constants are in hertz. Direct insertion probe chemical ionization mass spectral data were obtained on a Shimadzu GC-17A GC-MS mass spectrometer. Direct infusion electrospray ionization (in positively charged ion mode) mass spectral data were obtained on an Agilent 1100 series LC/MSD system (Germany). Melting points were determined on a Meltemp capillary melting point apparatus and were uncorrected. Infrared spectral data were obtained on a Perkin-Elmer Paragon 1000 FT-IR spectrophotometer. Optical rotation data was obtained from a Perkin-Elmer 241 polarimeter. The assigned structure of all test compounds and intermediates were consistent with the data. Carbon, hydrogen, and nitrogen elemental analyses for all novel targets were performed by Quantitative Technologies Inc., Whitehouse, NJ, and were within ± 0.4% of theoretical values except as noted; the presence of water or other solvents was confirmed by proton NMR. Reactions were generally performed in an argon or nitrogen atmosphere. Commercially purchased chemicals were used without purification unless otherwise noted. The following reagents were purchased from Aldrich Chemical Company: N-hydroxysuccinimide, phenethylamine, 3-phenyl-1-propylamine, 4-aminobiphenyl, palladium acetate, 4-phenylbenzylamine and benzyl amine. The following reagent was purchased from Trans World Chemicals: 2-(4-biphenyl ethylamine). The following reagents were purchased from Strem Chemicals, Incorporated: 1,1'-bis(diphenyl-phosphino)ferrocene (dppf) and dichloro[1,1'-bis(diphenylphosphino)-ferrocene]palladium (II) dichloromethane adduct [PdCl₂(dppf)]. Pyridine was distilled from KOH. DMF and DMSO were distilled over CaH₂ under reduced pressure. Silica gel (Bodman Industries, ICN SiliTech 2-63 D 60A, 230-400 Mesh) was used for all flash chromatography. Amines were purchased from Aldrich Chemical Company and used as received unless otherwise indicated. Toluene and Et₂O were distilled from sodium metal. THF was distilled from sodium/benzophenone ketyl. Pyridine was distilled from KOH. Methylene chloride was distilled from CaH₂. DMF and DMSO were distilled from CaH₂ under reduced pressure. Methanol was dried over 3± molecular sieves prior to use. Silica gel (Bodman Industries, ICN SiliTech 2-63 D 60A, 230-400 Mesh) was used for flash column chromatography.

In general, the chemistry described above works in the presence of the variety of functional groups found on known core structures. The exceptions would be morphine and congeners having a free 6-OH, which can be protected by a TBDPS (t-butyldiphenylsilyl) group [see Wentland et al., "Selective Protection and Functionalization of Morphine...", J. Med. Chem. 43, 3558-3565 (2000)].

## Claims

1. A compound of formula I: 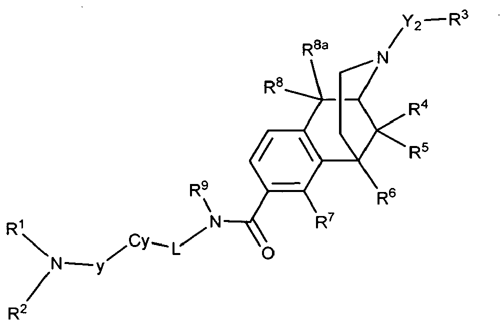 wherein
R¹ and R² are each independently chosen from hydrogen and optionally substituted lower alkyl;
R³ is chosen from hydrogen, C₁-C₈ hydrocarbon, heterocyclyl, aryl and hydroxyalkyl;
R⁴ is chosen from hydrogen, hydroxyl, C₁-C₂₀ alkyl and C₁-C₂₀ alkyl substituted with hydroxyl or carbonyl;
R⁵ is lower alkyl;
R⁶ is lower alkyl;
R⁷ is chosen from hydrogen, NR¹⁰R¹¹ and -OR¹⁰; or together R⁴, R⁵, R⁶ and R⁷ may form from one to three rings, said rings having optional additional substitution;
R⁸ and R^{8a} are both hydrogen or taken together R⁸ and R^{8a} are =O;
R⁹ is chosen from hydrogen and lower alkyl;
R¹⁰ and R¹¹ are each independently hydrogen, optionally substituted lower alkyl, optionally substituted alkenyl, optionally substituted alkynyl, optionally substituted aryl, hydroxyl, amino or optionally substituted lower alkoxy;
y is -(C(R¹⁰)(R¹¹))p- or a direct bond, wherein p is 0, 1, 2, 3, 4, 5, 6, or 7;
Y₂ is a direct bond or -(C(R¹⁰)(R¹¹))q-, wherein q is 0, 1, 2, 3, 4 or 5;
L is a direct bond or -(C(R¹⁰)(R¹¹))q-; and
Cy is Ar¹-B-Ar², wherein
Ar¹ is absent, or an aryl or heteroaryl radical having from 1 to 4 N, O and/or S atoms, which may be unsubstituted or mono-, di- or trisubstituted by halogen, lower alkyl, alkenyl, alkynyl, cycloalkyl, -OR¹⁰, -NR¹⁰R¹¹, -CN, -COR¹⁰ or - COOR¹⁰;
B is a direct bond, -O-, -NR¹⁰, -SO₂, or -(C(R¹¹)(R¹¹))s-, wherein s is 0, 1, 2, 3, 4 or 5; and
Ar² is aryl or heteroaryl radical having from 1 to 4 N, O and/or S atoms, which may be unsubstituted or mono-, di- or trisubstituted by halogen, lower alkyl, alkenyl, alkynyl, cycloalkyl, -OR¹⁰, -NR¹⁰R¹¹, -CN, -COR¹⁰ or -COOR¹⁰,
wherein optionally substituted alkyl, alkenyl, alkynyl, aryl, or alkoxy refers to alkyl, alkenyl, aryl, or alkoxy wherein optionally up to three H atoms in each residue are replaced with halogen, haloalkyl, alkyl, acyl, alkoxyalkyl, hydroxyloweralkyl, phenyl, heteroaryl, benzenesulfonyl, hydroxy, loweralkoxy, haloalkoxy, carboxy, carboalkoxy (also referred to as alkoxycarbonyl), alkoxycarbonylamino, carboxamido (also referred to as alkylaminocarbonyl), cyano, carbonyl, acetoxy, nitro, amino, alkylamino, dialkylamino, mercapto, alkylthio, sulfoxide, sulfone, sulfonylamino, acylamino, amidino, aryl, benzyl, heterocyclyl, phenoxy, benzyloxy, heteroaryloxy, hydroxyimino, alkoxyimino, oxaalkyl, aminosulfonyl, trityl, amidino, guanidino, and ureido; and
wherein lower alkyl refers to alkyl groups of from 1 to 6 carbon atoms; alkyl refers to alkyl groups of C₂₀ or below; cycloalkyl includes cyclic hydrocarbon groups of from 3 to 8 carbon atoms; alkoxy or alkoxyl refers to groups of from 1 to 8 carbon atoms; lower-alkoxy refers to groups containing one to four carbons; aryl and heteroaryl mean a 5- or 6-membered aromatic or heteroaromatic ring containing 0-3 heteroatoms selected from O, N, or S; a bicyclic 9- or 10-membered aromatic or heteroaromatic ring system containing 0-3 heteroatoms selected from O, N, or S; or a tricyclic 13- or 14-membered aromatic or heteroaromatic ring system containing 0-3 heteroatoms selected from O, N, or S; wherein one or more rings are aromatic, but not all need be; and C, to C₂₀ hydrocarbon means a linear, branched, or cyclic residue comprised of hydrogen and carbon as the only elemental constituents and includes alkyl, cycloalkyl, polycycloalkyl, alkenyl, alkynyl, aryl and combinations thereof.

2. A compound of claim 1 wherein Cy is:

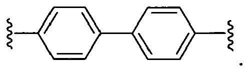

3. A compound of claim 1 of formula II: 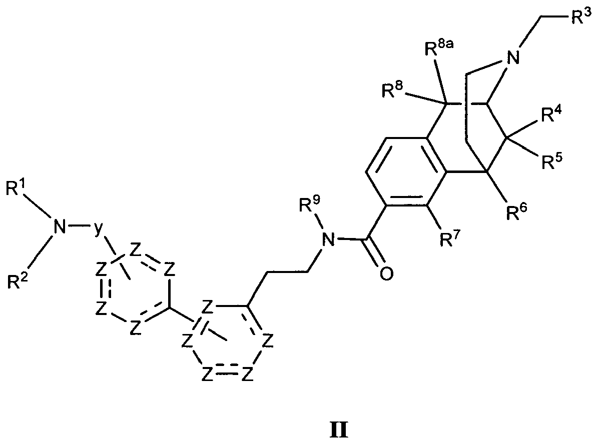 wherein
Z is CR¹⁰ or N, with the proviso that,
at the points of attachment of the NR¹R²y group to the distal aromatic ring and of the distal aromatic ring to the proximal aromatic ring, Z must be C,
wherein preferably the compound is of formula

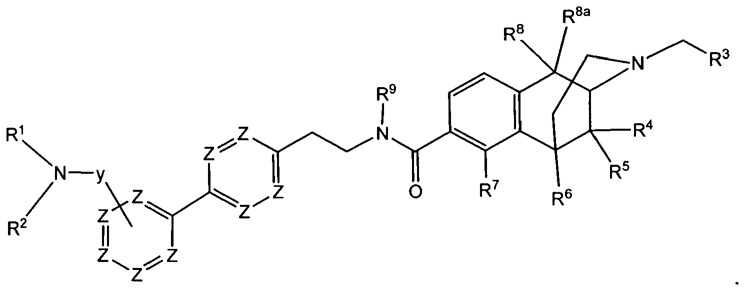

4. A compound according to claim 3 of formula

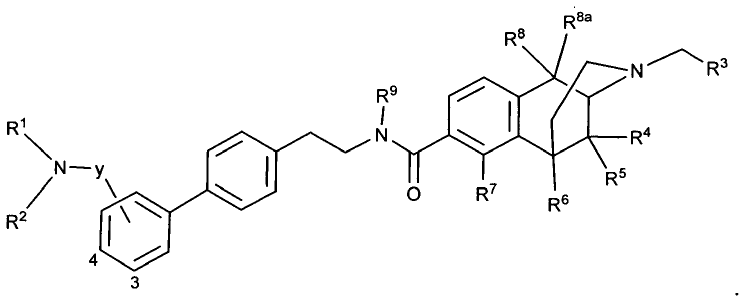

5. A compound according to any of claims 1-4 wherein:
R³ is chosen from hydrogen, cyclopropyl, cyclobutyl, phenyl, vinyl, dimethylvinyl, hydroxycyclopropyl, furanyl, and tetrahydrofuranyl;
R⁴ is chosen from hydrogen and 3-oxo-5-cyclopentyl-1-pentanyl;
R⁵ is methyl;
R⁶ is methyl or ethyl;
R⁸ and R^{8a} are both hydrogen; and
R⁹ is hydrogen.

6. A compound according to claim 5, wherein -yNR¹R² is substituted at the 4-position.

7. A compound according to claim 6, wherein y is a direct bond or CH₂.

8. A compound according to claim 5, wherein -yNR¹R² is substituted at the 3-position.

9. A compound according to claim 8, wherein y is a direct bond.

10. A compound according to claim 7 or 9, wherein R¹ and R² are each selected from methyl and hydrogen or R¹ is hydrogen and R² is substituted alkyl

11. A compound according to claim 1, wherein together R⁵ and R⁶ form one ring, said compound having the structure: 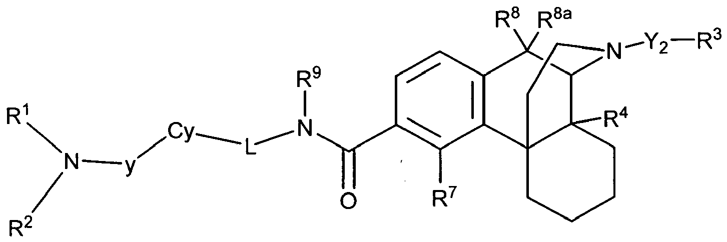 wherein preferably:
R⁸ and R^{8a} are hydrogen;
R³ is chosen from hydrogen, cyclopropyl, cyclobutyl, vinyl and tetrahydrofuranyl; and
R⁴ is hydrogen or hydroxyl.

12. A compound according to claim 1, wherein together R⁵, R⁶ and R⁷ form two rings, having the structure: 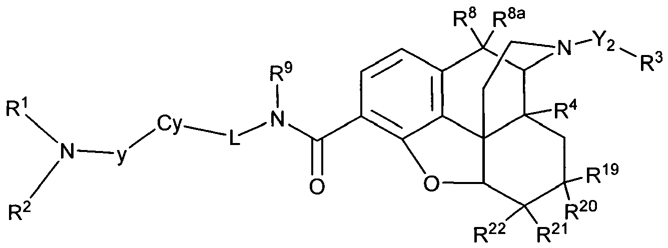 wherein
R⁴ is hydrogen or hydroxy;
R¹⁹ is hydrogen or lower alkyl;
R²⁰ is chosen from hydrogen, lower alkyl and hydroxy(lower alkyl); or together, R¹⁹ and R²⁰ form a spiro-fused carbocycle of 5 to 10 carbons;
R²¹ is hydrogen;
R²² is chosen from hydroxy, lower alkoxy and -NR¹³R¹⁴; or together, R²¹ and R²² form a carbonyl or a vinyl substituent;
R¹³ is hydrogen or optionally substituted lower alkoxy; and
R¹⁴ is hydrogen, optionally substituted lower alkoxy, acyl or fumarate,
wherein preferably
together, R⁴ and R²¹ form a sixth ring, of formula: 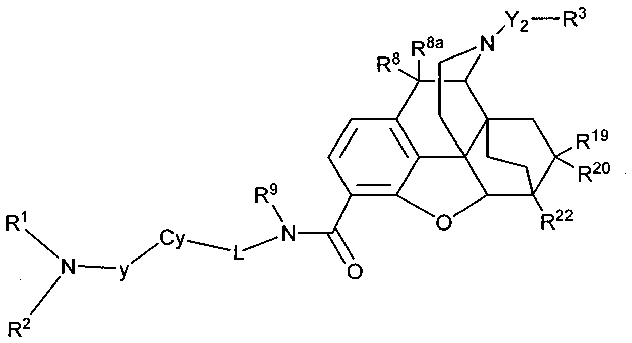 or
R⁴ and R²¹ form a sixth ring, of formula 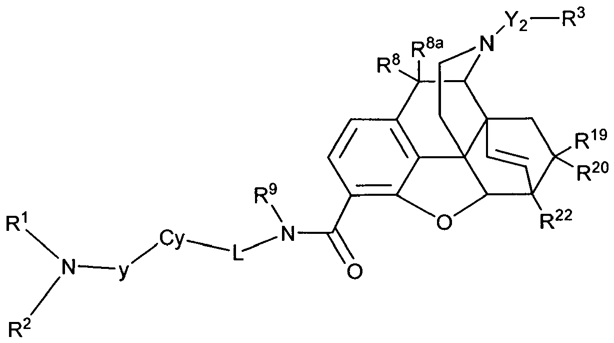 wherein
R¹⁹ is hydrogen;
R²⁰ is hydroxy(lower alkyl); and
R²² is lower alkoxy.

13. A compound according to claim 11 or 12 wherein 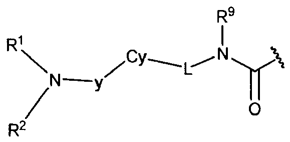 is represented by

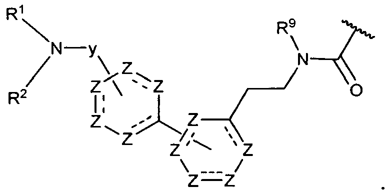

14. A compound of claim 1 selected from: 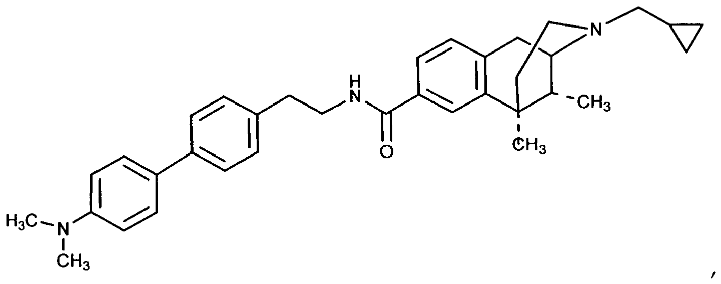 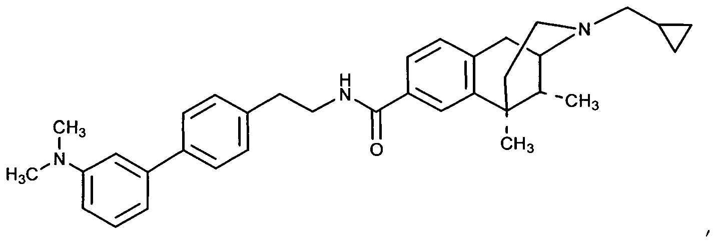 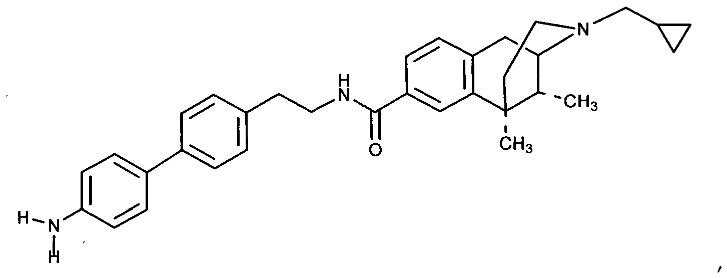 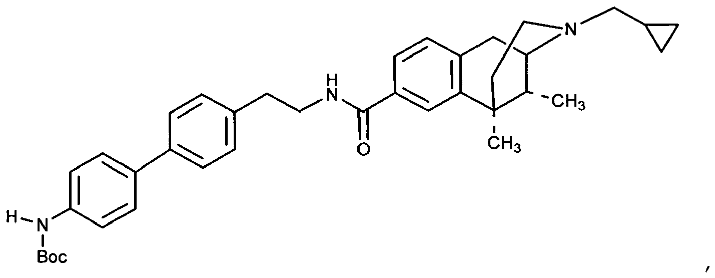 and

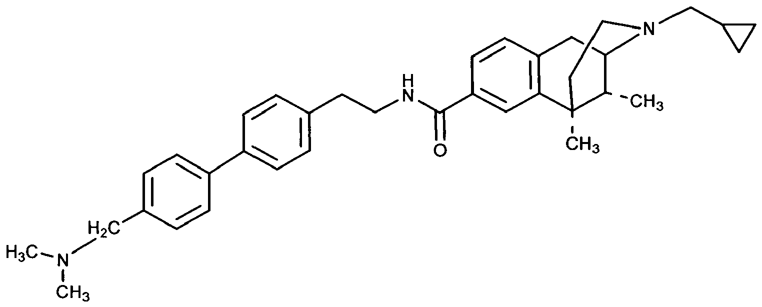

15. A pharmaceutical formulation comprising a compound according to any of claims 1-14 and a pharmaceutically acceptable carrier.

16. A compound according to any one of claims 1-14 for use in preventing or treating a condition or disease associated with binding opioid receptors in a patient in need thereof, wherein said disease or condition is chosen from the group consisting of pain, pruritis, diarrhea, irritable bowel syndrome, gastrointestinal motility disorder, obesity, respiratory depression, convulsions, coughing, hyperalgesia and drug addiction, wherein preferably said drug addiction is selected from heroin, cocaine, nicotine and alcohol addiction.

## Patentansprüche

1. Eine Verbindung der Formel I: 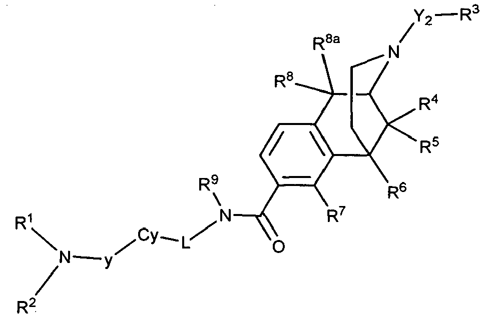 wobei
R¹ und R² jeweils unabhängig voneinander ausgewählt sind aus Wasserstoff und optional substituiertem niederem Alkyl;
R³ ausgewählt ist aus Wasserstoff, C₁-C₈ Kohlenwasserstoff, Heterocyclyl, Aryl und Hydroxyalkyl;
R⁴ ausgewählt ist aus Wasserstoff, Hydroxyl, C₁-C₂₀ Alkyl und C₁-C₂₀ Alkyl substituiert mit Hydroxyl oder Carbonyl;
R⁵ niederes Alkyl ist;
R⁶ niederes Alkyl ist;
R⁷ ausgewählt ist aus Wasserstoff, NR¹⁰R¹¹ und -OR¹⁰; oder R⁴, R⁵, R⁶ und R⁷ gemeinsam einen bis drei Ringe bilden können, wobei besagte Ringe optional zusätzlich Substitution aufweisen;
R⁸ und R^{8a} beide Wasserstoff oder R⁸ und R^{8a} gemeinsam =O sind;
R⁹ ausgewählt ist aus Wasserstoff und niederem Alkyl;
R¹⁰ und R¹¹ jeweils unabhängig voneinander Wasserstoff, optional substituiertes niederes Alkyl, optional substituiertes Alkenyl, optional substituiertes Alkynyl, optional substituiertes Aryl, Hydroxyl, Amino oder optional substituiertes niederes Alkoxy sind;
y -(C(R¹⁰)(R¹¹))p- oder eine direkte Bindung ist, wobei p 0, 1, 2, 3, 4, 5, 6, oder 7 ist;
Y₂ eine direkte Bindung oder -(C(R¹⁰)(R¹¹))q- ist, wobei q 0, 1, 2, 3, 4 oder 5 ist;
L eine direkte Bindung oder -(C(R¹⁰)(R¹¹))q- ist; und
Cy Ar¹-B-Ar² ist, wobei
Ar¹ nicht vorhanden oder ein Aryl- oder Heteroaryl-Radikal, aufweisend von 1 bis 4 N, O und/oder S-Atomen, ist, die unsubstituiert oder mit Halogen, niederem Alkyl, Alkenyl, Alkynyl, Cycloalkyl, -OR¹⁰, -NR¹⁰R¹¹, -CN, -COR¹⁰ oder -COOR¹⁰mono-, di- oder trisubstituiert sein können;
B eine direkte Bindung, -O-, -NR¹⁰, -SO₂, oder -(C(R¹⁰)(R¹¹))s- ist, wobei s 0, 1, 2, 3, 4 oder 5 ist; und
Ar² ein Aryl- oder Heteroaryl-Radikal, aufweisend von 1 bis 4 N, O und/oder S-Atomen, ist, die unsubstituiert oder mit Halogen, niederem Alkyl, Alkenyl, Alkynyl, Cycloalkyl, -OR¹⁰, -NR¹⁰R¹¹, -CN, -COR¹⁰ oder -COOR¹⁰ mono-, di-oder trisubstituiert sein können,
wobei optional substituiertes Alkyl, Alkenyl, Alkynyl, Aryl, oder Alkoxy sich auf Alkyl, Alkenyl, Aryl oder Alkoxy bezieht, wobei optional bis zu drei H-Atome an jedem Rest durch Halogen, Haloalkyl, Alkyl, Acyl, Alkoxyalkyl, Hydroxy-Niederalkyl, Phenyl, Heteroaryl, Benzensulfonyl, Hydroxy, niederes Alkoxy, Haloalkoxy, Carboxy, Carboalkoxy (auch als Alkoxycarbonyl bezeichnet), Alkoxycarbonylamino, Carboxamido (auch als Alkylaminocarbonyl bezeichnet), Cyano, Carbonyl, Acetoxy, Nitro, Amino, Alkylamino, Dialkylamino, Mercapto, Alkylthio, Sulfoxid, Sulfon, Sulfonylamino, Acylamino, Amidino, Aryl, Benzyl, Heterocyclyl, Phenoxy, Benzyloxy, Heteroaryloxy, Hydroxyimino, Alkoxyimino, Oxaalkyl, Aminosulfonyl, Trityl, Amidino, Guanidino und Ureido ersetzt werden; und
wobei sich niederes Alkyl auf Alkyl-Gruppen von 1 bis 6 Kohlenstoffatomen bezieht; Alkyl sich auf Alkyl-Gruppen von C₂₀ oder weniger bezieht; Cycloalkyl zyklische Kohlenwasserstoffgruppen von 3 bis 8 Kohlenstoffen beinhaltet; Alkoxy oder Alkoxyl sich auf Gruppen von 1 bis 8 Kohlenstoffatomen bezieht; niederes Alkoxy sich auf Gruppen, enthaltend ein bis vier Kohlenstoffe bezieht; Aryl und Heteroaryl einen 5-oder 6-gliedrigen aromatischen oder heteroaromatischen Ring bezeichnen, enthaltend 0-3 Heteroatome, augewählt aus O, N, oder S; ein bizyklisches 9- oder 10-gliedriges aromatisches oder heteroaromatisches Ringsystem, enthaltend 0-3 Heteroatome, ausgewählt aus O, N, oder S; oder ein trizyklisches 13- oder 14-gliedriges aromatisches oder heteroaromatisches Ringsystem, enthaltend 0-3 Heteroatome, ausgewählt aus O, N, oder S; wobei ein oder mehrere Ringe aromatisch sind, aber nicht alle derart sein müssen; und C₁ bis C₂₀ Kohlenwasserstoff einen linearen, verzweigten oder zyklischen Rest bezeichnen, umfassend Wasserstoff und Kohlenstoff als die einzigen elementaren Konstituenten, und Alkyl, Cycloalkyl, Polycycloalkyl, Alkenyl, Alkynyl, Aryl und Kombinationen hiervon einschließen.

2. Eine Verbindung nach Anspruch 1, wobei Cy 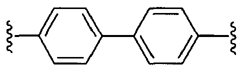 ist.

3. Eine Verbindung nach Anspruch 1 der Formel II: 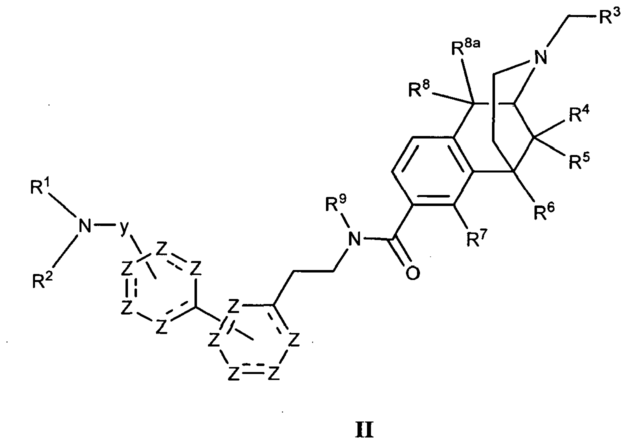 wobei
Z CR¹⁰ oder N ist, mit der Bedingung, dass
an den Verbindungsstellen der NR¹R²y-Gruppe zum distalen aromatischen Ring und von dem distalen aromatischen Ring zum proximalen aromatischen Ring Z C sein muss,
wobei die Verbindung vorzugsweise der Formel 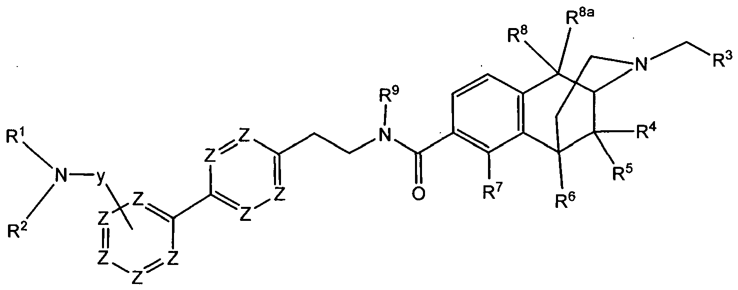 entspricht.

4. Eine Verbindung gemäß Anspruch 3 der Formel

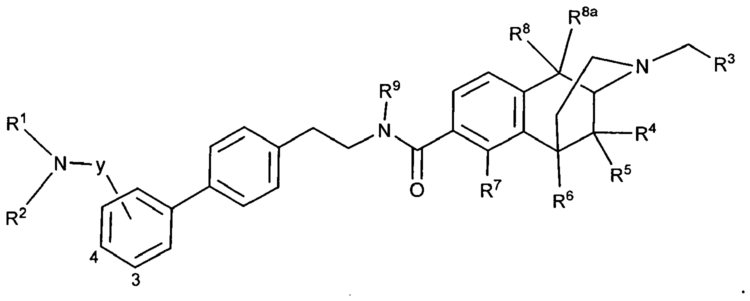

5. Eine Verbindung gemäß einem der Ansprüche 1 bis 4, wobei:
R³ aus Wasserstoff, Cyclopropyl, Cyclobutyl, Phenyl, Vinyl, Dimethylvinyl, Hydroxycyclopropyl, Furanyl und Tetrahydrofuranyl ausgewählt ist;
R⁴ aus Wasserstoff und 3-Oxo-5-Cyclopentyl-1-Pentanyl ausgewählt ist;
R⁵ Methyl ist;
R⁶ Methyl oder Ethyl ist;
R⁸ und R^{8a} beide Wasserstoff sind; und
R⁹ Wasserstoff ist.

6. Eine Verbindung gemäß Anspruch 5, wobei -yNR¹R² an der 4-Position substituiert ist.

7. Eine Verbindung gemäß Anspruch 6, wobei y eine direkte Bindung oder CH₂ ist.

8. Eine Verbindung gemäß Anspruch 5, wobei -yNR¹R² an der 3-Position substituiert ist.

9. Eine Verbindung gemäß Anspruch 8, wobei y eine direkte Bindung ist.

10. Eine Verbindung gemäß Anspruch 7 oder 9, wobei R¹ und R² jeweils aus Methyl und Wasserstoff ausgewählt sind oder R¹ Wasserstoff und R² substituiertes Alkyl ist.

11. Eine Verbindung gemäß Anspruch 1, wobei R⁵ und R⁶ gemeinsam einen Ring bilden, wobei besagte Verbindung die Struktur aufweist: 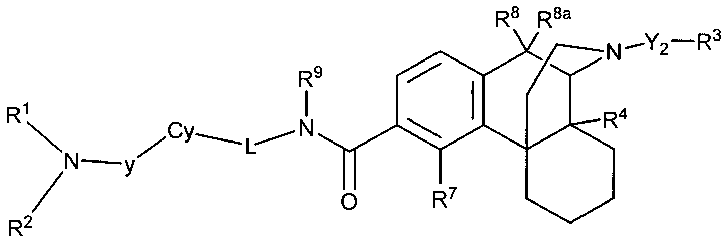 wobei vorzugsweise:
R⁸ und R^{8a} Wasserstoff sind;
R³ ausgewählt ist aus Wasserstoff, Cyclopropyl, Cyclobutyl, Vinyl und Tetrahydrofuranyl; und
R⁴ Wasserstoff oder Hydroxyl ist.

12. Eine Verbindung gemäß Anspruch 1, wobei R⁵, R⁶ und R⁷ gemeinsam zwei Ringe bilden mit der Struktur: 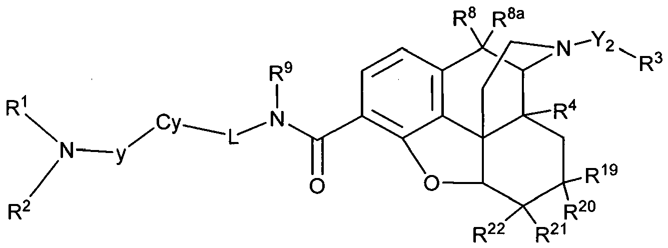 wobei
R⁴ Wasserstoff oder Hydroxy ist;
R¹⁹ Wasserstoff oder niederes Alkyl ist;
R²⁰ aus Wasserstoff, niederem Alkyl und Hydroxy-(Niederalkyl) ausgewählt ist; oder R¹⁹ und R²⁰ gemeinsam einen Spiro-fusionierten Kohlenstoffzyklus von 5 bis 10 Kohlenstoffen bilden;
R²¹ Wasserstoff ist;
R²² aus Hydroxy, niederem Alkoxy und -NR¹³R¹⁴ ausgewählt ist; oder R²¹ und R²² gemeinsam einen Carbonyl- oder einen Vinyl-Substituenten bilden;
R¹³ Wasserstoff oder optional substituiertes niederes Alkoxy ist; und
R¹⁴ Wasserstoff, optional substituiertes niederes Alkoxy, Acyl oder Fumarat ist,
wobei vorzugsweise
R⁴ und R²¹ gemeinsam einen sechsten Ring bilden, gemäß der Formel: 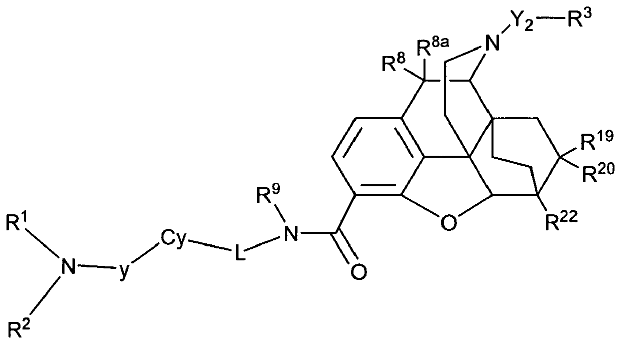 oder
R⁴ und R²¹ einen sechsten Ring bilden, gemäß der Formel 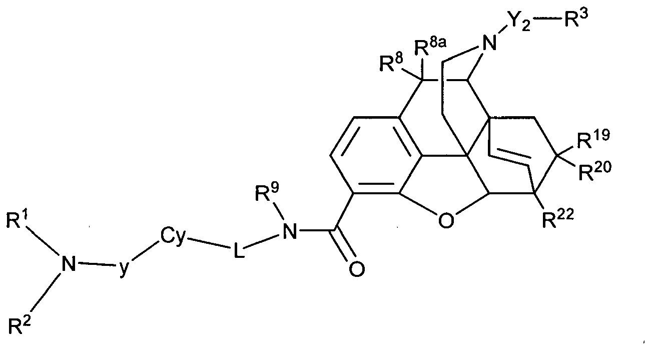 wobei
R¹⁹ Wasserstoff ist;
R²⁰ Hydroxy-(Niederalkyl) ist; und
R²² niederes Alkoxy ist.

13. Eine Verbindung gemäß Anspruch 11 oder 12, wobei 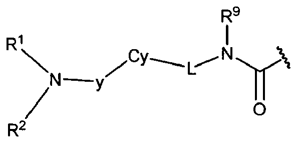 dargestellt ist durch

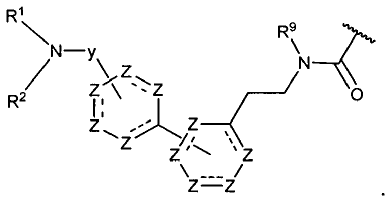

14. Eine Verbindung nach Anspruch 1, ausgewählt aus: 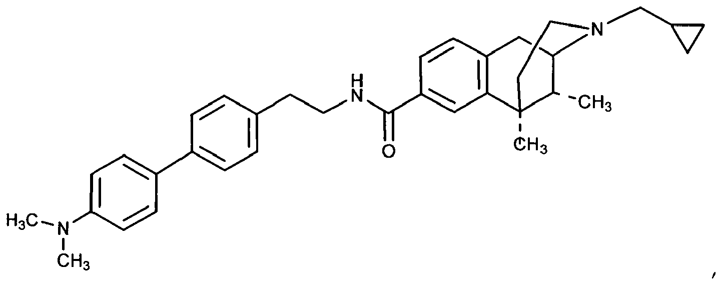 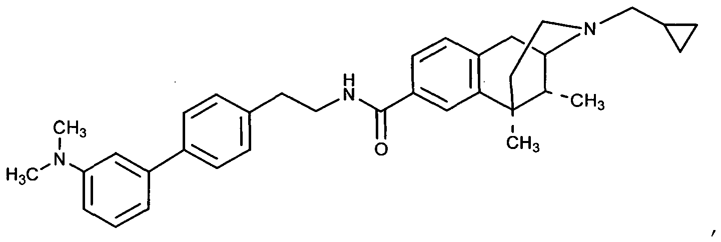 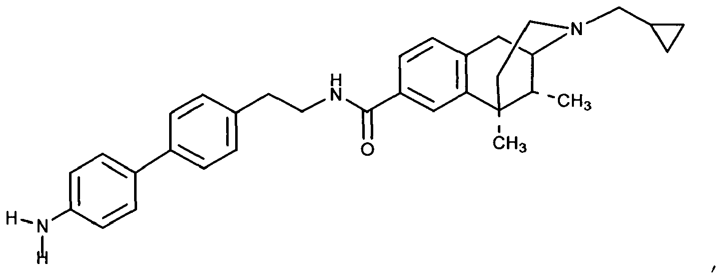 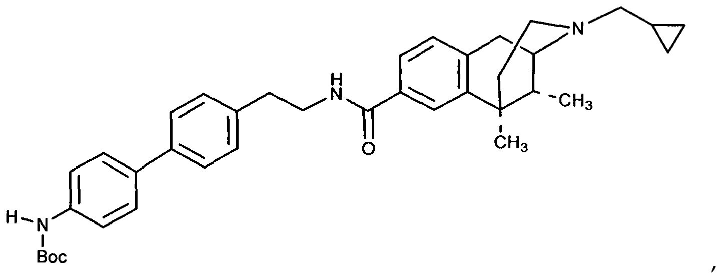 und

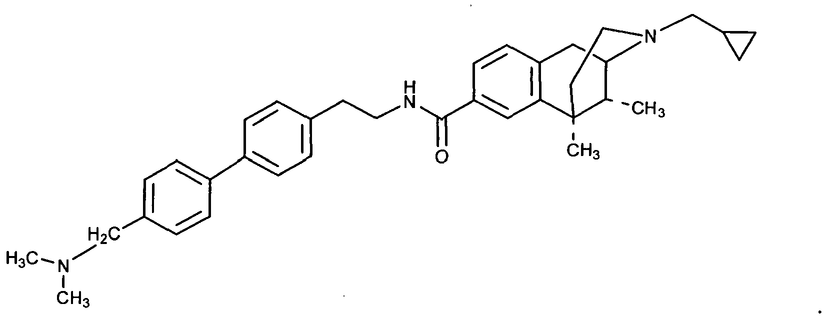

15. Eine pharmazeutische Formulierung, umfassend eine Verbindung gemäß einem der Ansprüche 1-14 und einen pharmazeutisch akzeptablen Träger.

16. Eine Verbindung gemäß einem der Ansprüche 1-14 für die Verwendung zur Vorbeugung oder Behandlung eines Zustands oder einer Erkrankung, assoziiert mit der Bindung an Opioid-Rezeptoren, in einem Patienten mit Bedarf hierfür, wobei besagte Erkrankung oder besagter Zustand ausgewählt ist aus der Gruppe bestehend aus Schmerz, Juckreiz, Diarrhöe, Reizdarmsyndrom, gastrointestinaler Motilitätsstörung, Fettleibigkeit, Atemdepression, Krämpfen, Husten, Hyperalgesie und Drogensucht, wobei besagte Drogensucht vorzugsweise aus Heroin-, Kokain-, Nikotin- und Alkoholsucht ausgewählt ist.

## Revendications

1. Composé de formule I : 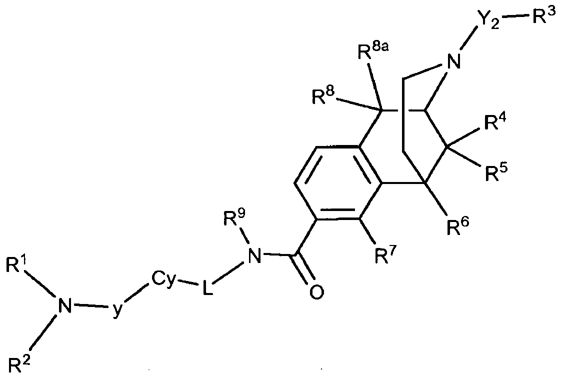 dans laquelle
chacun de R¹ est R² est indépendamment choisi parmi l'hydrogène et les radicaux alkyle inférieur éventuellement substitués ;
R³ est choisi parmi l'hydrogène et les radicaux hydrocarbonés en C₁ à C₈, hétérocyclyle, aryle et hydroxyalkyle ;
R⁴ est choisi parmi l'hydrogène et les radicaux hydroxyle, alkyle en C₁ à C₂₀ et alkyle en C₁ à C₂₀ substitués par hydroxyle ou carbonyle ;
R⁵ est un radical alkyle inférieur ;
R⁶ est un radical alkyle inférieur ;
R⁷ est choisi parmi l'hydrogène, NR¹⁰R¹¹ et -OR¹⁰; ou bien
R⁴, R⁵, R⁶ et R⁷ peuvent former ensemble d'un à trois cycles, lesdits cycles portant une substitution additionnelle éventuelle ;
R⁸ et R^{8a} sont tous deux l'hydrogène ou, pris ensemble, R⁸ et R^{8a} forment =O ;
R⁹ est choisi parmi l'hydrogène et les radicaux alkyle inférieur ;
chacun de R¹⁰ et R¹¹ est indépendamment l'hydrogène ou un radical alkyle inférieur éventuellement substitué, alcényle éventuellement substitué, alcynyle éventuellement substitué, aryle éventuellement substitué, hydroxyle, amino, ou alcoxy inférieur éventuellement substitué ;
y est -(C(R¹⁰)(R¹¹))p- ou une liaison directe, où p vaut 0, 1, 2, 3, 4, 5, 6 ou 7 ;
Y₂ est une liaison directe ou -(C(R¹⁰)(R¹¹))q-, où q vaut 0, 1, 2, 3, 4 ou 5 ;
L est une liaison directe ou -(C(R¹⁰)(R¹¹))q- ; et
Cy est Ar¹-B-Ar², où
Ar¹ est absent ou est un radical aryle ou hétéroaryle ayant 1 à 4 atomes N, O et/ou S, qui peut être non substitué ou mono-, di- ou tri-substitué par un halogène ou un radical alkyle inférieur, alcényle, alcynyle, cycloalkyle, - OR¹⁰, -NR¹⁰R¹¹, -CN, -COR¹⁰ ou -COOR¹⁰;
B est une liaison directe, -O-, -NR¹⁰, -SO₂, ou -(C(R¹⁰)(R¹¹))s- où s vaut 0, 1, 2, 3, 4 ou 5 ; et
Ar² est un radical aryle ou hétéroaryle ayant 1 à 4 atomes N, O et/ou S, qui peut être non substitué ou mono-, di- ou tri-substitué par un halogène ou un radical alkyle inférieur, alcényle, alcynyle, cycloalkyle, -OR¹⁰, -NR¹⁰R¹¹, - CN, -COR¹⁰ ou -COOR¹⁰,
où le radical éventuellement substitué alkyle, alcényle, alcynyle, aryle ou alcoxy se réfère à un radical alkyle, alcényle, aryle ou alcoxy dans lequel éventuellement jusqu'à trois atomes H dans chaque résidu sont remplacés par des atomes d'halogène ou des radicaux halogénoalkyle, alkyle, acyle, alcoxyalkyle, hydroxyalkyle inférieur, phényle, hétéroaryle, benzènesulfonyle, hydroxy, alcoxy inférieur, halogénoalcoxy, carboxy, carbalcoxy (également appelé alcoxycarbonyle), alcoxycarbonylamino, carboxamido (également appelé alkylaminocarbonyle), cyano, carbonyle, acétoxy, nitro, amino, alkylamino, dialkylamino, mercapto, alkylthio, sulfoxyde, sulfone, sulfonylamino, acylamino, amidino, aryle, benzyle, hétérocyclyle, phénoxy, benzyloxy, hétéroaryloxy, hydroxyimino, alcoxyimino, oxalkyle, aminosulfonyle, trityle, amidino, guanidino, ou uréido ; et
où l'alkyle inférieur se réfère à des groupes alkyle ayant de 1 à 6 atomes de carbone ; l'alkyle se réfère à des groupes alkyle en C₂₀ ou moins ; le cycloalkyle englobe les groupes hydrocarbonés cycliques ayant de 3 à 8 atomes de carbone ; l'alcoxy ou alcoxyle se réfère à des groupes ayant de 1 à 8 atomes de carbone ; l'alcoxy inférieur se réfère à des groupes contenant un à quatre carbones ; l'aryle et l'hétéroaryle signifient un cycle aromatique ou hétéroaromatique à 5 ou 6 chaînons contenant 0-3 hétéroatomes choisis parmi O, N et S ; un système cyclique aromatique ou hétéroaromatique bicyclique à 9 ou 10 chaînons contenant 0-3 hétéroatomes choisis parmi O, N et S ; ou un système cyclique aromatique ou hétéroaromatique tricyclique à 13 ou 14 chaînons contenant 0-3 hétéroatomes choisis parmi O, N et S ; où un ou plusieurs cycles sont aromatiques, mais tous n'ont pas besoin de l'être ; et l'hydrocarbure en C₁ à C₂₀ signifie un résidu linéaire, ramifié ou cyclique constitué d'hydrogène et de carbone en tant que seuls éléments constitutifs et englobe les radicaux alkyle, cycloalkyle, polycycloalkyle, alcényle, alcynyle, aryle, et leurs combinaisons.

2. Composé selon la revendication 1, dans lequel Cy est :

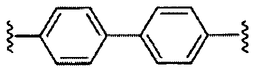

3. Composé selon la revendication 1, de formule II : 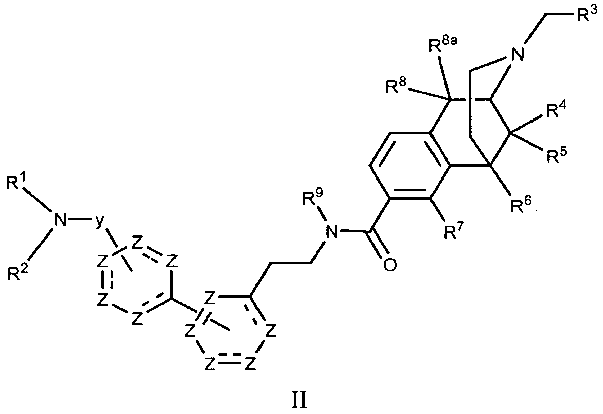 dans laquelle
Z est CR¹⁰ ou N, à la condition suivante :
aux points de rattachement du groupe NR¹R²y au cycle aromatique distal et du cycle aromatique distal au cycle aromatique proximal, Z doit être C,
lequel composé est de préférence de formule

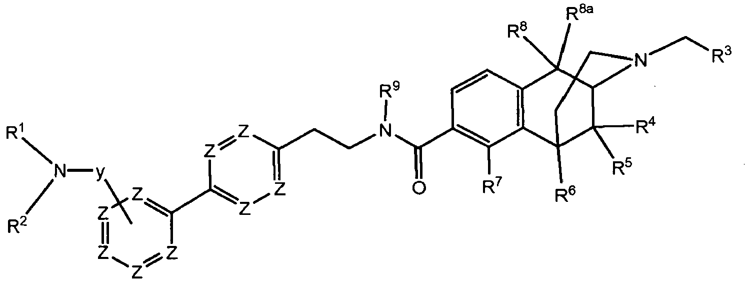

4. Composé selon la revendication 3, de formule :

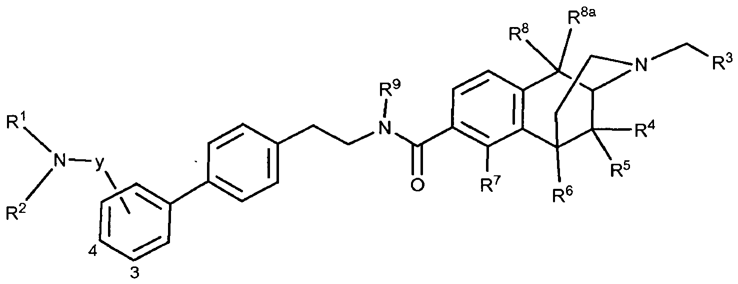

5. Composé selon l'une quelconque des revendications 1 à 4, dans lequel:
R³ est choisi parmi l'hydrogène et les radicaux cyclopropyle, cyclobutyle, phényle, vinyle, diméthylvinyle, hydroxycyclopropyle, furanyle, et tétrahydrofuranyle ;
R⁴ est choisi parmi l'hydrogène et le radical 3-oxo-5-cyclopentyl-1-pentanyle ;
R⁵ est le radical méthyle ;
R⁶ est le radical méthyle ou éthyle ;
R⁸ et R^{8a} sont tous deux des hydrogènes ; et
R⁹ est l'hydrogène.

6. Composé selon la revendication 5, dans lequel -yNR¹R² est substitué en position 4.

7. Composé selon la revendication 6, dans lequel y est une liaison directe ou CH₂.

8. Composé selon la revendication 5, dans lequel -yNR¹R² est substitué en position 3.

9. Composé selon la revendication 8, dans lequel y est une liaison directe.

10. Composé selon la revendication 7 ou 9, dans lequel chacun de R¹ et R² est choisi parmi le radical méthyle et l'hydrogène, ou bien R¹ est l'hydrogène et R² est un radical alkyle substitué.

11. Composé selon la revendication 1, dans lequel R⁵ et R⁶ forment ensemble un seul cycle, ledit composé ayant la structure : 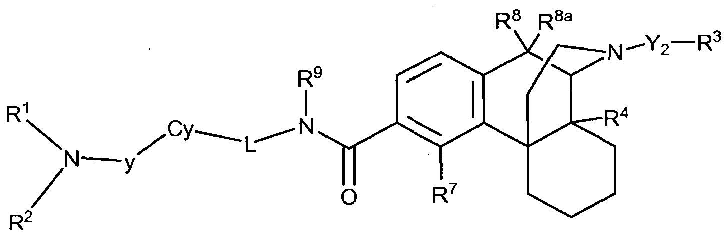 dans laquelle, de préférence :
R⁸ et R^{8a} sont des hydrogènes ;
R³ est choisi parmi l'hydrogène et les radicaux cyclopropyle, cyclobutyle, vinyle et tétrahydrofuranyle ; et
R⁴ est l'hydrogène ou le radical hydroxyle.

12. Composé selon la revendication 1, dans lequel R⁵, R⁶ et R⁷ forment ensemble deux cycles, ayant la structure : 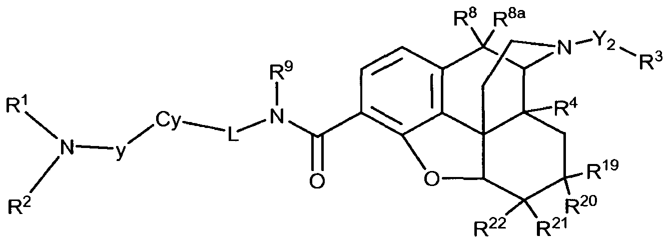 dans laquelle
R⁴ est l'hydrogène ou le radical hydroxy ;
R¹⁹ est l'hydrogène ou un radical alkyle inférieur ;
R²⁰ est choisi parmi l'hydrogène et les radicaux alkyle inférieur et hydroxyalkyle inférieur ;
ou bien R¹⁹ et R²⁰ forment ensemble un carbocycle spiro-condensé ayant 5 à 10 carbones ;
R²¹ est l'hydrogène ;
R²² est choisi parmi les radicaux hydroxy, alcoxy inférieur et -NR¹³R¹⁴;
ou bien R²¹ et R²² forment ensemble un radical carbonyle ou un substituant vinyle ; R¹³ est l'hydrogène ou un radical alcoxy inférieur éventuellement substitué ; et
R¹⁴ est l'hydrogène ou un radical alcoxy inférieur éventuellement substitué, acyle ou fumarate,
de préférence dans laquelle R⁴ et R²¹ forment un sixième cycle, de formule : 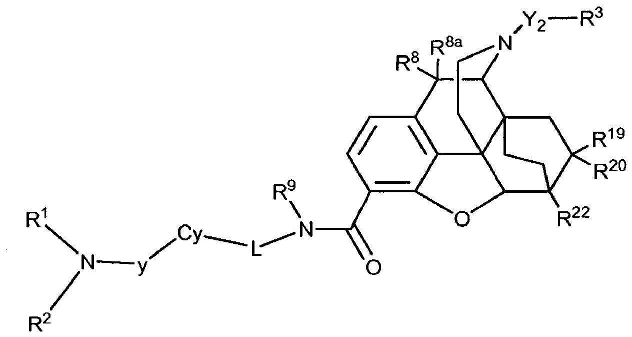
ou bien R⁴ et R²¹ forment un sixième cycle, de formule : 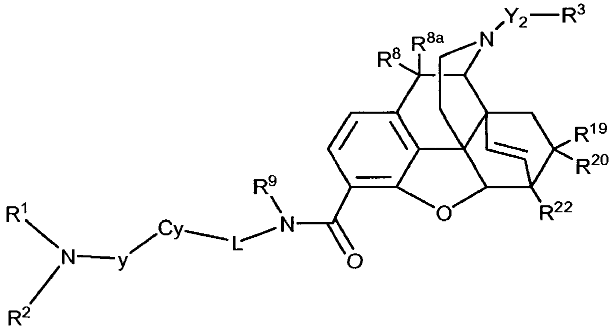 dans laquelle
R¹⁹ est l'hydrogène ;
R²⁰ est un radical hydroxyalkyle inférieur ; et
R²² est un radical alcoxy inférieur.

13. Composé selon la revendication 11 ou 12, dans lequel 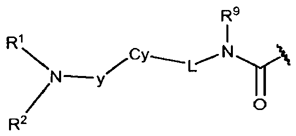 est représenté par

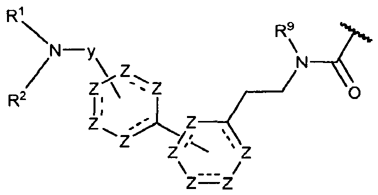

14. Composé selon la revendication 1, choisi parmi : 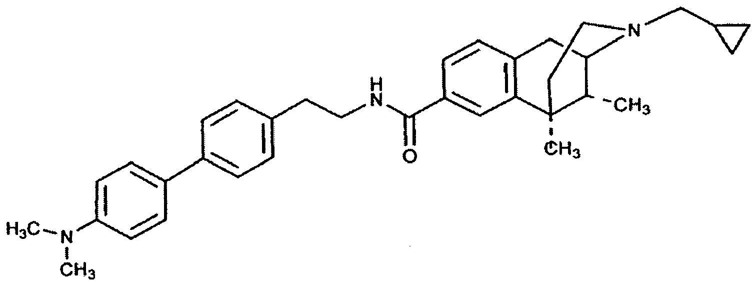 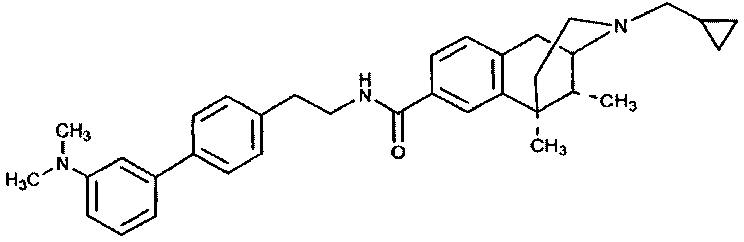 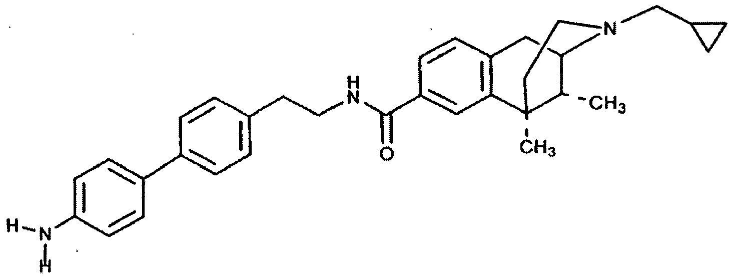 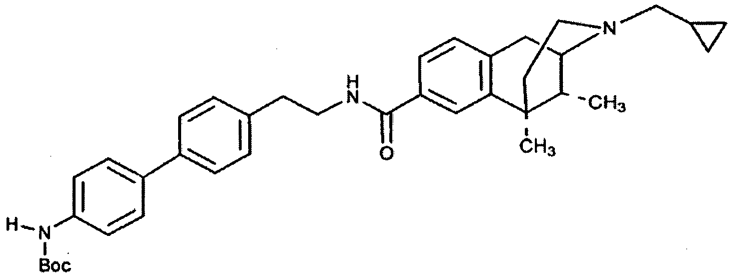 et

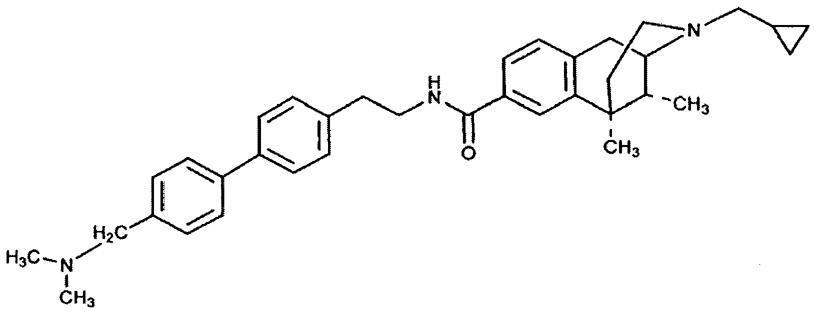

15. Formulation pharmaceutique comprenant un composé selon l'une quelconque des revendications 1 à 14 et un véhicule pharmaceutiquement acceptable.

16. Composé selon l'une quelconque des revendications 1 à 14, pour son utilisation dans la prévention ou le traitement d'une condition ou d'une maladie associée à la liaison de récepteurs d'opioïdes chez un patient en ayant besoin, dans lequel ladite maladie ou condition est choisie dans le groupe constitué par une douleur, un prurit, une diarrhée, un syndrome du côlon irritable, un trouble de la motilité gastro-intestinale, l'obésité, une dépression respiratoire, des convulsions, une toux, une hyperalgésie et une pharmacodépendance, dans lequel de préférence ladite pharmacodépendance est choisie parmi la dépendance à l'héroïne, la cocaïne, la nicotine et l'alcool.
